(19) Europäisches Patentamt
European Patent Office
Office européen des brevets

(11) **EP 3 118 324 A1**

(12) **EUROPEAN PATENT APPLICATION**

(43) Date of publication:
**18.01.2017 Bulletin 2017/03**

(51) Int Cl.:
*C12Q 1/68* (2006.01)   *G06F 19/18* (2011.01)
*G06F 19/20* (2011.01)   *G06F 19/22* (2011.01)

(21) Application number: **15176404.0**

(22) Date of filing: **13.07.2015**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA**

(71) Applicant: **Cartagenia N.V.
3001 Leuven (BE)**

(72) Inventors:
• **Allemeersch, Joke
3200 Aarschot (BE)**
• **Devogelaere, Benoit
1800 Vilvoorde (BE)**

(74) Representative: **Brantsandpatents bvba
Pauline Van Pottelsberghelaan 24
9051 Ghent (BE)**

(54) **METHOD FOR ANALYZING COPY NUMBER VARIATION IN THE DETECTION OF CANCER**

(57)    The current invention concerns a method for identifying the presence of a cancer and/or an increased risk of a cancer in a mammal, by calculating a parameter obtained from a biological sample whereby said cutoff value is a prerequisite for the presence or absence of one or more aneuploidies in said target chromosome or chromosome segment which is an indicator of the presence and/or increased risk of cancer.

EP 3 118 324 A1

**Description**

**Technical field**

The invention pertains to the technical field of a method for determining copy number variations (CNV) of a sequence of interest in a test sample that comprises a mixture of nucleic acids that are known or are suspected to differ in the amount of one or more sequence of interest. The method comprises a statistical approach that accounts for accrued variability stemming from process-related, inter-chromosomal and inter-sequencing variability. The method is applicable to determining CNVs known or suspected to be associated with a variety of medical conditions. CNVs that can be determined according to the method include trisomies and monosomies of any one or more of chromosomes 1-22, X and Y, other chromosomal nullisomies and polysomies, and deletions and/or duplications and/or amplifications of segments of any one or more of the chromosomes, which can be detected by sequencing the nucleic acids of a test sample.

**Background**

One of the critical endeavors in human medical research is the discovery of genetic abnormalities that produce adverse health consequences. In many cases, specific genes and/or critical diagnostic markers have been identified in segments of the genome that are present at abnormal copy numbers. For example, in prenatal diagnosis, extra or missing copies of whole chromosomes are frequently occurring genetic abnormalities In cancer, deletion or amplification of copies of whole chromosomes or chromosomal segments or specific regions of the genome, are common occurrences.
Most information about copy number variation has been provided by cytogenetic resolution that has permitted recognition of structural abnormalities. Conventional procedures for genetic screening and biological dosimetry have utilized invasive procedures e.g. amniocentesis or solid tumor biopsies, to obtain cells for the analysis of karyotypes. Recognizing the need for more rapid testing methods that do not require cell culture, fluorescence in situ hybridization (FISH), quantitative fluorescence PCR (QF-PCR) and array-Comparative Genomic Hybridization (array-CGH) have been developed as molecular-cytogenetic methods for the analysis of copy number variations.
The advent of technologies that allow for sequencing entire genomes in relatively short time, and the discovery of circulating cell-free DNA (cfDNA) have provided the opportunity to compare genetic material originating from one chromosome to be compared to that of another without the risks associated with invasive sampling methods.
US20130034546 and US20130310263 both describe a method for identifying the presence of cancer or the risk to develop the latter based on an analysis of obtained sequence reads obtained from a cell free DNA fraction in a sample.
Vandenberghe et al., "Non-invasive detection of genomic imbalances in Hodgkin/Reed-Sternberg cells in early and advanced stage Hodgkin's lymphoma by sequencing of circulating cell-free DNA: a technical proof-of-principle study", 2015 describes a methodology for identifying genomic imbalances in a presymptomatic Hodgkin lymphoma cancer patient by massive parallel sequencing of circulating cell-free DNA.
However, the limitations of the existing methods, which include insufficient sensitivity stemming from the limited levels of cfDNA, and the sequencing bias of the technology stemming from the inherent nature of genomic information, underlie the continuing need for noninvasive methods that would provide any or all of the specificity, sensitivity, and applicability, to reliably diagnose copy number changes in a variety of clinical settings.
Although above mentioned methodologies have their value, the ratio of false positives and negatives remain high in the field. It is therefore a constant striving to provide methodologies that are able to lower the percentage of false positives and especially false negatives, in order to provide more accurate screening.
The current invention wishes to provide a more accurate, essentially non-invasive methodology for determining whether an individual has tumor-derived cell-free DNA in his or her peripheral blood, for confirming a cancer diagnosis, for aiding in the classification of a cancer, for assessing the treatment response, for monitoring the patient..

**Summary of the invention**

The present invention provides a method, systems and an apparatus for determining whether a nucleic acid sequence imbalance (e.g., chromosome imbalance or an imbalance of a chromosome segment or region) or a genome-wide instability exists within a biological sample obtained from a subject or for determining copy number variations.
In particular to current invention provides for a method according to claim 1 and a computer program product according to claim 27.
Other embodiments of the invention are directed to systems and computer readable media associated with methods described herein.

**DESCRIPTION OF FIGURES**

**Figure 1** shows a plot of secondary parameters obtained according to an embodiment of the current invention per chromosome from a sample.

**Figure 2** shows histograms of chromosomes from a sample, whereby the calculation of a parameter according to an embodiment of the current invention indicates genome-wide instability which could be suggestive for the presence of a tumor.

**DEFINITIONS**

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

As used herein, the following terms have the following meanings:

The term "biological sample" as used herein refers to any sample that is taken from a subject (e.g., a human, such as a pregnant woman) and contains one or more nucleic acid molecule(s) of interest.

The term "nucleic acid" or "polynucleotide" refers to a deoxyribonucleic acid (DNA) or ribonucleic acid (RNA) and a polymer thereof in either single- or double-stranded form. Unless specifically limited, the term encompasses nucleic acids containing known analogs of natural nucleotides that have similar binding properties as the reference nucleic acid and are metabolized in a manner similar to naturally occurring nucleotides. Unless otherwise indicated, a particular nucleic acid sequence also implicitly encompasses conservatively modified variants thereof (e.g., degenerate codon substitutions), alleles, orthologs, Single Nucleotide Polymorphisms (SNPs), and complementary sequences as well as the sequence explicitly indicated. Specifically, degenerate codon substitutions may be achieved by generating sequences in which the third position of one or more selected (or all) codons is substituted with mixed-base and/or deoxyinosine residues. The term nucleic acid is used interchangeably with gene, DNA, cDNA, mRNA, small noncoding RNA, micro RNA (miRNA), Piwi-interacting RNA, and short hairpin RNA (shRNA) encoded by a gene or locus.

The term "gene" means the segment of DNA involved in producing a polypeptide chain. It may include regions preceding and following the coding region (leader and trailer) as well as intervening sequences (introns) between individual coding segments (exons).

The term "reaction" as used herein refers to any process involving a chemical, enzymatic, or physical action that is indicative of the presence or absence of a particular polynucleotide sequence of interest. An example of a "reaction" is an amplification reaction such as a polymerase chain reaction (PCR). Another example of a "reaction" is a sequencing reaction, either by synthesis, hybridization or by passing the DNA through a pore and measuring signals that are indicative for a particular nucleotide. An "informative reaction" is one that indicates the presence of one or more particular polynucleotide sequence of interest, and in one case where only one sequence of interest is present. The term "well" as used herein refers to a reaction at a predetermined location within a confined structure, e.g., a well-shaped vial, cell, or chamber in a PCR array or e.g. the individual reaction volumes in which sequencing reactions take place (thereby including so-called patterned flow cells from Illumina).

The term "clinically relevant nucleic acid sequence" or "target chromosome or chromosomal segment" as used herein can refer to a polynucleotide sequence corresponding to a segment of a larger genomic sequence whose potential imbalance is being tested or to the larger genomic sequence itself. One example is the sequence of chromosome 21. Other examples include chromosome 18, 13, X and Y. Yet other examples include mutated genetic sequences or genetic polymorphisms or copy number variations (CNVs) that a fetus may inherit from one or both of its parents. Yet other examples include sequences which are mutated, deleted, or amplified in a malignant tumor, e.g. sequences in which loss of heterozygosity or gene duplication occur. In some embodiments, multiple clinically relevant nucleic acid sequences, or equivalently multiple makers of the clinically relevant nucleic acid sequence, can be used to provide data for detecting the imbalance. For instance, data from five non-consecutive sequences on chromosome 21 can be used in an additive fashion for the determination of possible chromosomal 21 imbalance, effectively reducing the need of sample volume to 1/5.

The term "overrepresented nucleic acid sequence" as used herein refers to the nucleic acid sequence among two sequences of interest (e.g., a clinically relevant sequence and a background sequence) that is in more abundance than the other sequence in a biological sample.

The term "based on" as used herein means "based at least in part on" and refers to one value (or result) being used in the determination of another value, such as occurs in the relationship of an input of a method and the output of that method. The term "derive" as used herein also refers to the relationship of an input of a method and the output of that method, such as occurs when the derivation is the calculation of a formula.

The term "parameter" herein refers to a numerical value that characterizes a quantitative data set and/or a numerical relationship between quantitative data sets. For example, a ratio (or function of a ratio) between the number of sequence reads mapped to a chromosome and the length of the chromosome to which the reads are mapped, is a parameter.

The term "score" as used herein refers to a numerical value which is linked or based on a specific feature, e.g. the number of reads or read counts of a certain sequence present in a sample. The term "first score" is used herein to refer to a numerical value linked to the target chromosome or chromosomal segment. Another example of a score is e.g. a Z score that quantifies how much the number of reads of a certain sequence differs from the number of reads that were obtained from the same sequence in a set of reference samples. It is known to a person skilled in the art how such a Z score can be calculated. The term "cutoff value" or "threshold" as used herein means a numerical value whose value is used to arbitrate between two or more states (e.g. diseased and non-diseased) of classification for a biological sample. For example, if a parameter is greater than the cutoff value, a first classification of the quantitative data is made (e.g. diseased state); or if the parameter is less than the cutoff value, a different classification of the quantitative data is made (e.g. non-diseased state).

The term "imbalance" as used herein means any significant deviation as defined by at least one cutoff value in a quantity of the clinically relevant nucleic acid sequence from a reference quantity. For example, the reference quantity could be a ratio of 3/5, and thus an imbalance would occur if the measured ratio is 1:1.

The term "random sequencing" as used herein refers to sequencing whereby the nucleic acid fragments sequenced have not been specifically identified or targeted before the sequencing procedure. Sequence-specific primers to target specific gene loci are not required. The pools of nucleic acids sequenced vary from sample to sample and even from analysis to analysis for the same sample. The identities of the sequenced nucleic acids are only revealed from the sequencing output generated. In some embodiments of the present invention, the random sequencing may be preceded by procedures to enrich a biological sample with particular populations of nucleic acid molecules sharing certain common features. In one embodiment, each of the DNA fragments in the biological sample have an equal probability of being sequenced.

The term "fraction of the human genome" or "segment of the human genome" as used herein refers to less than 100% of the nucleotide sequences in the human genome which comprises of some 3 billion basepairs of nucleotides. In the context of sequencing, it refers to less than 1-fold coverage of the nucleotide sequences in the human genome. The term may be expressed as a percentage or absolute number of nucleotides/basepairs. As an example of use, the term may be used to refer to the actual amount of sequencing performed. Embodiments may determine the required minimal value for the sequenced fraction of the human genome to obtain an accurate diagnosis. As another example of use, the term may refer to the amount of sequenced data used for deriving a parameter or amount for disease classification.

The term "summary statistics" as used herein is to be understood as a statistical term, and is to be understood as an indication of the extend of a distribution of values or scores, or as indication of the score/value present in the middle of the distribution. This can be e.g. a mean or median or standard deviation (StDev) or median absolute deviation (mad) or mean absolute deviation of a collection of scores.

The term "copy number variation" or "CNV" herein refers to variation in the number of copies of a nucleic acid sequence that is a few bp kb or larger present in a test sample in comparison with the copy number of the nucleic acid sequence present in a qualified sample. A "copy number variant" refers to the a few bp or larger sequence of nucleic acid in which copy-number differences are found by comparison of a sequence of interest in test sample with that present in a qualified sample. Copy number variants/variations include deletions, including microdeletions as well as amplifications. CNVs encompass chromosomal aneuploidies and partial aneuplodies.

The term "aneuploidy" herein refers to an imbalance of genetic material caused by a loss or gain of a whole chromosome, or part of a chromosome. Aneuploidy refers to both chromosomal as well as subchromosomal imbalances, such as, but not limiting to deletions, microdeletions, insertions, microinsertions, copy number variations, duplications. The Copy Number Variations may vary in size in the range of 1 kb to multiple Mb. Large subchromosomal abnormalities that span a region of tens of MBs and/or correspond to a significant portion of a chromosome arm, can also be referred to as segmental aneuploidies.

The term "chromosomal aneuploidy" herein refers to an imbalance of genetic material caused by a loss or gain of a whole chromosome, and includes germline aneuploidy and mosaic aneuploidy.

The term "partial aneuploidy" herein refers to an imbalance of genetic material caused by a loss or gain of a part of a chromosome e.g. partial monosomy and partial trisomy, and encompasses imbalances resulting from translocations, deletions and insertions.

The terms "polymorphism, polymorphic target nucleic acid", "polymorphic sequence", "polymorphic target nucleic acid sequence" and "polymorphic nucleic acid" are used interchangeably herein to refer to a nucleic acid sequence that contains one or more polymorphic sites.

The term "polymorphic site" herein refers to a single nucleotide polymorphism (SNP), a small-scale multi-base deletion or insertion, a Multi-Nucleotide Polymorphism (MNP) or a Short Tandem Repeat (STR) or a CNV (copy number variation).

The term "plurality" is used herein in reference to a number of nucleic acid molecules or sequence tags or reads that is sufficient to identify significant differences in copy number variations (e.g. chromosome doses) in test samples and qualified samples using the methods of the invention. In some embodiments, at least about $3 \times 10^6$ sequence tags, at least about $5 \times 10^6$ sequence tags, at least about $8 \times 10^6$ sequence tags, at least about $10 \times 10^6$ sequence tags, at least about $15 \times 10^6$ sequence tags, at least about $20 \times 10^6$ sequence tags, at least about $30 \times 10^6$ sequence tags, at least about $40 \times 10^6$ sequence tags, or at least about $50 \times 10^6$ sequence tags are obtained for each test sample. Each sequence tag can be a single sequence read of 20 to 400 bp, or a couple of 2 paired-end sequence reads of each 20 to 400 bp.

The terms "polynucleotide", "nucleic acid" and "nucleic acid molecules" are used interchangeably and refer to a covalently linked sequence of nucleotides (i.e., ribonucleotides for RNA and deoxyribonucleotides for DNA) in which the 3' position of the pentose of one nucleotide is joined by a phosphodiester group to the 5' position of the pentose of the next, include sequences of any form of nucleic acid, including, but not limited to RNA and DNA molecules. The term "polynucleotide" includes, without limitation, single- and double-stranded polynucleotide.

The term "portion" when used in reference to the amount of sequence information of nucleic acid molecules in a biological sample herein refers to the amount of sequence information of nucleic acid molecules in a biological sample that in sum amount to less than the sequence information of < 1 human genome.

The term "test sample" herein refers to a sample comprising a mixture of nucleic acids comprising at least one nucleic acid sequence whose copy number is suspected of having undergone variation or at least one nucleic acid sequence for which it is desired to determine whether a copy number variation exists. Nucleic acids present in a test sample are referred to as "test nucleic acids" or target nucleic acids or target chromosomes or target chromosomal segments..

The term "reference sample" herein refers to a sample comprising a mixture of nucleic acids from which the sequencing data are used along with the test sample sequencing data to calculate scores and parameters as described in claim 1. Though not necessary, a reference sample is preferably normal i.e. not aneuploid, for the sequence of interest. So preferably, a reference sample is a qualified sample that does not carry a trisomy 21 and that can be used for identifying for the presence of a trisomy 21 in a test sample.

The term "reference set" comprises a plurality of "reference samples".

The term "enrich" herein refers to the process of specifically amplifying certain target nucleic acids contained in a segment of a sample. The amplified product is then often combined with the remainder of the sample from which the segment was removed.

The term "sequence of interest" herein refers to a nucleic acid sequence that is associated with a difference in sequence representation in healthy versus diseased individuals. A sequence of interest can be a sequence on a chromosome that is misrepresented i.e. over- or under-represented, in a disease or genetic condition. A sequence of interest may also be a segment of a chromosome, or a chromosome. For example, a sequence of interest can be a chromosome that is over-represented in an aneuploidy condition, or a gene encoding a tumor-suppressor that is under-represented in a cancer. Sequences of interest include sequences that are over- or under-represented in the total population, or a subpopulation of cells of a subject.

The term "plurality of polymorphic target nucleic acids" herein refers to a number of nucleic acid sequences each comprising at least one polymorphic site e.g. one SNP or CNV, such that at least 1, 2, 3, 4, 5, 6, 7, 8, 9, 10, 15, 20, 25, 30, 40 or more different polymorphic sites are amplified.

The term "group of chromosomes" herein refers to two or more chromosomes. The term "collection" refers to a set of chromosomes or chromosomal segments, but may also refer to a set of values or scores derived from a corresponding set of chromosomes or chromosomal segments.

The term "read" refers to an experimentally obtained DNA sequence of sufficient length (e.g., at least about 20 bp) that can be used to identify a larger sequence or region, e.g. that can be aligned and specifically assigned to a chromosome location or genomic region or gene.

The term "read count" refers to the number of reads retrieved from a sample that are mapped to a reference genome or a segment of said reference genome (bin). The term "bin" of a genome is to be understood as a segment of the genome. A genome can be divided in several bins, either of a fixed or predetermined size or a variable size. A possible bin size can be e.g. 10 kB, 20 kB, 30 kB, 40 kB, 50 kB, 60 kB, 70 kB, etc.

The term "window" is to be understood as a plurality of bins.

The terms "aligned", "alignment", "mapped" or "aligning", "mapping" refer to one or more sequences that are identified as a match in terms of the order of their nucleic acid molecules to a known sequence from a reference genome. Such alignment can be done manually or by a computer algorithm, examples including the Efficient Local Alignment of Nucleotide Data (ELAND) computer program distributed as part of the Illumina Genomics Analysts pipeline. The matching of a sequence read in aligning can be a 100% sequence match or less than 100% (non-perfect match).

The term "reference genome" as used herein refers to a digital nucleic acid sequence database, assembled as a representative example of a species' DNA. As it is assembled from the sequencing of DNA from multiple, a reference genome does not accurately represent the DNA of a single person. It is used to enable the mapping of sequencing reads from a sample to specific chromosomal positions.

The term "clinically-relevant sequence" herein refers to a nucleic acid sequence that is known or is suspected to be associated or implicated with a genetic or disease condition. Determining the absence or presence of a clinically-relevant sequence can be useful in determining a diagnosis or confirming a diagnosis of a medical condition, or providing a prognosis for the development of a disease.

The term "derived" when used in the context of a nucleic acid or a mixture of nucleic acids, herein refers to the means whereby the nucleic acid(s) are obtained from the source from which they originate. For example, in one embodiment, a mixture of nucleic acids that is derived from two different genomes means that the nucleic acids e.g. cell-free DNA, were naturally released by cells through naturally occurring processes such as necrosis or apoptosis, or through lysis of the cells due to improper storage or transport conditions.

The term "biological fluid" herein refers to a liquid taken from a biological source and includes, for example, blood, serum, plasma, sputum, lavage fluid, cerebrospinal fluid, urine, semen, sweat, tears, saliva, blastocoel fluid and the like. It also refers to the medium in which biological samples can be grown, like in vitro culture medium in which cells, tissue or embryo can be cultured. As used herein, the terms "blood," "plasma" and "serum" expressly encompass fractions or processed segments thereof. Similarly, where a sample is taken from a biopsy, swab, smear, etc., the "sample" expressly encompasses a processed fraction or segment derived from the biopsy, swab, smear, etc.

The term "corresponding to" herein refers to a nucleic acid sequence e.g. a gene or a chromosome, that is present in the genome of different subjects, and which does not necessarily have the same sequence in all genomes, but serves to provide the identity rather than the genetic information of a sequence of interest e.g. a gene or chromosome.

The term "substantially cell free" herein refers to preparations of the desired sample from which components that are normally associated with it are removed. For example, a plasma sample is rendered essentially cell free by removing blood cells e.g. white blood cells, which are normally associated with it. In some embodiments, substantially free samples are processed to remove cells that would otherwise contribute to the genetic material that is to be tested for an aneuploidy.

As used herein the term "chromosome" refers to the heredity-bearing gene carrier of a living cell which is derived from chromatin and which comprises DNA and protein components (especially histones). The conventional internationally recognized individual human genome chromosome numbering system is employed herein. The term "chromosomal segments" is to be understood as a part of a chromosome. Said segment may refer to a bin, window or specific region within a chromosome, e.g. known to comprise for instance deletions or insertions or copy number variations.

As used herein, the term "polynucleotide length" refers to the absolute number of nucleic acid molecules (nucleotides) in a sequence or in a region of a reference genome. The term "chromosome length" refers to the known length of the chromosome given in base pairs.

The term "subject" herein refers to a human subject as well as a non-human subject such as a mammal, an invertebrate, a vertebrate, a fungus, a yeast, a bacteria, and a virus. Although the examples herein concern human cells and the language is primarily directed to human concerns, the concept of this invention is applicable to genomes from any plant or animal, and is useful in the fields of veterinary medicine, animal sciences, research laboratories and such.

The term "condition" herein refers to "medical condition" as a broad term that includes all diseases and disorders, but can include injuries and normal health situations, such as pregnancy, that might affect a person's health, benefit from medical assistance, or have implications for medical treatments. By preference, said condition is linked to the presence of a tumor.

## Detailed description of the invention

The present invention concerns method for determining whether a subject has tumor-derived cell-free DNA in his or her peripheral blood, for confirming a cancer diagnosis, for aiding in the classification of a cancer, for assessing the treatment response, for monitoring the subject, for identifying the presence of a cancer and/or an increased risk of a cancer in a subject, said subject is preferably a mammal. This identification may be done by the calculation of a parameter linked to chromosomal and/or subchromosomal data obtained from a biological sample. Also provided is a computer readable medium encoded with a plurality of instructions for controlling a computer system to perform the methods.

In one aspect, an amount of read counts linked to a chromosome or chromosomal segment is determined from a sequencing of nucleic acid molecules in a biological sample, such as urine, plasma, serum, blastocoel fluid and other suitable biological samples. Nucleic acid molecules of the biological sample are randomly sequenced, such that a fraction of the genome is sequenced. One or more cutoff values are chosen for determining whether a change compared to a reference quantity exists (i.e. an imbalance), for example, with regards to the ratio of amounts of two chromosomal regions (or sets of regions).

The target chromosomal region (also called a clinically relevant nucleic acid sequence) and the background nucleic acid sequence may come from a first type of cells and from one or more second types of cells.

I. General method for evaluating an aneuploidy

The current invention describes a methodology for determining whether a subject has tumor-derived cell-free DNA in his or her peripheral blood, for confirming a cancer diagnosis, for aiding in the classification of a cancer, for assessing the treatment response, for monitoring the subject, for identifying the presence of a cancer and/or an increased risk of a cancer in a subject, said subject is preferably a mammal.

In a first aspect, the method for determining whether a subject has tumor-derived cell-free DNA in his or her peripheral blood, for confirming a cancer diagnosis, for aiding in the classification of a cancer, for assessing the treatment response, for monitoring the subject, for identifying the presence of a cancer and/or an increased risk of a cancer in a subject is based on the determination of a parameter from the nucleic acid content of a biological sample. The biological sample may be plasma, urine, serum, blastocoel fluid or any other suitable sample. For example, the nucleic acid molecules may be fragments from chromosomes.

At least a portion of a plurality of the nucleic acid molecules contained in the biological sample is randomly sequenced to obtain a number of sequences. The portion sequenced represents a fraction of the human genome and may be isolated from the sample by conventional means (e.g. cell-free DNA extraction means and preparation of a NGS library). In one embodiment, the nucleic acid molecules are fragments of respective chromosomes. One end (e.g. 50 basepairs (bp)), both ends, or the entire fragment may be sequenced. A subset of the nucleic acid molecules in the sample may be sequenced, and this subset is randomly chosen, as will be described in more detail later.

In one embodiment, the random sequencing is done using massively parallel sequencing. Massively parallel sequencing, such as that achievable on the HiSeq2500, HiSeq3000, HiSeq4000, HiSeq X, MiSeq, MiSeqDx, NextSeq500, NextSeq550 flowcell, the 454 platform (Roche), Illumina Genome Analyzer (or Solexa platform) or PGM or Proton platform (IonTorrent) or GeneRead (Qiagen) or SOLiD System (Applied Biosystems) or the Helicos True Single Molecule DNA sequencing technology, the single molecule, real-time (SMRT™) technology of Pacific Biosciences, and nanopore sequencing as in MinION, PrmethION, GridION (Oxford Nanopore technologies), allow the sequencing of many nucleic acid molecules isolated from a specimen at high orders of multiplexing in a parallel fashion. Each of these platforms sequences clonally expanded or even non-amplified single molecules of nucleic acid fragments. Clonal expansion can be achieved via bridge amplification, emulsion PCR, or Wildfire technology.

As a high number of sequencing reads, in the order of hundred thousand to millions or even possibly hundreds of millions or billions, are generated from each sample in each run, the resultant sequenced reads form a representative profile of the mix of nucleic acid species in the original specimen. For example, the haplotype, transcriptome and methylation profiles of the sequenced reads resemble those of the original specimen. Due to the large sampling of sequences from each specimen, the number of identical sequences, such as that generated from the sequencing of a nucleic acid pool at several folds of coverage or high redundancy, is also a good quantitative representation of the count of a particular nucleic acid species or locus in the original sample.

Based on the sequencing (e.g. data from the sequencing), a first score of a target chromosome or chromosomal segment is determined. The first score is determined from sequences identified as originating from (i.e. aligning) to the target chromosome or segment. For example, a bioinformatics procedure may then be used to locate each of these DNA sequences to the human genome or a reference genome. It is possible that a proportion of such sequences will be discarded from subsequent analysis because they are present in the repeat regions of the human genome, or in regions subjected to inter-individual variations, e.g. copy number variations. A score of the target chromosome or chromosomal segment and of one or more other chromosomes may thus be determined.

Based on the sequencing, a collection of scores of one or more chromosomes or chromosomal segments is determined from sequences identified as originating from (i.e. aligning to) a set of one of more chromosomes. In one embodiment, said set contains all of the other chromosomes besides the first one (i.e. the one being tested). In another embodiment, said set contains just a single other chromosome. In a most preferred embodiment, said set contains chromosomes or chromosomal segments and includes the target chromosome or chromosomal segment.

There are a number of ways of determining a score. By preference, said score is based on the read counts obtained from sequencing. Said read counts can include, but are not limiting to counting the number of reads, the number of sequenced nucleotides (basepairs) or the accumulated lengths of sequenced nucleotides (basepairs) originating from particular chromosome(s) or chromosomal segments such as bins or windows or clinically relevant chromosome portions.

Rules may be imposed on the results of the sequencing to determine what gets counted. In one aspect, a read count may be obtained based on a proportion of the sequenced output. For example, sequencing output corresponding to nucleic acid fragments of a specified size range could be selected.

In one embodiment, said score is the raw read count for a certain chromosome or chromosomal segment.

In a preferred embodiment, said read counts are subjected to mathematical functions or operations in order to derive said score of said read counts. Such operations include but are not limiting to statistical operations, regression models standard calculations (sum, subtraction, multiplying and division), whereby said standard calculations are preferably based on one or more obtained read counts.

In a preferred embodiment, said first score is a normalized value derived from the read counts or mathematically modified read counts. In a further preferred embodiment, said score is a Z score or standard score relating to the read counts of a certain chromosome, chromosomal segment or the mathematically amended counts, in which the Z score quantifies how much the number of reads of a certain sequence differs from the number of reads that were obtained from the same sequence in a set of reference samples. It is known to a person skilled in the art how such a Z score can be calculated.

In a preferred embodiment, a parameter is determined based on a first score (corresponding to the target chromosome or chromosomal segment) and a collection of scores. The parameter preferably represents a relative score between the first score and a summary statistic of the collection of scores. The parameter may be, for example, a simple ratio of the first score to a summary statistic of the collection of scores. In one aspect, each score could be an argument to a function or separate functions, where a ratio may be then taken of these separate functions. The parameter may be, for example, a simple ratio of the first score to a summary statistic of scores present in the collection. In one aspect, each score could be an argument to a function or separate functions, where a ratio may be then taken of these separate functions.

In a preferred embodiment, the parameter may be obtained by a ratio between:

- a first function whereby the first score and the collection of scores are the arguments;

- a second function whereby the collection of scores is the argument.

In a more preferred embodiment, said first function is defined as a difference, preferably the difference between the first score and a summary statistic of the collection of scores, whereby said summary statistic is preferably selected from the mean, median, standard deviation or median absolute deviation (mad) or mean absolute deviation.

In a further preferred embodiment, said second function is defined as a variability summary statistic of the collection of scores, whereby said summary statistic may be an average or a measure of variability and is preferably selected from the mean, median, standard deviation or median absolute deviation (mad) or mean absolute deviation.

Typically, a suitable embodiment according to the current invention involves the following steps (after having obtained DNA sequences from a random, low-coverage sequencing process on a biological sample).

- aligning sequences to a reference genome;

- obtaining the read counts per chromosome or chromosomal segment;

- normalizing the number of reads or a derivative thereof towards a normalized number of reads;

- obtaining a first score derived from said normalized number of reads and a collection of scores derived from said normalized reads, whereby said first score is derived from the normalized read counts for a target chromosome or chromosomal segment , and said collection of scores is a set of scores derived from the normalized number of reads that were obtained from a set of chromosomes or chromosome segments that include the target chromosomal segment or chromosome;

- calculating a parameter from said scores, whereby said parameter represents a ratio between said first score and a summary statistic of said collection of scores, whereby the first function of said ratio is defined as a difference between the first score and a summary statistic of said collection of scores; and whereby the second function of said ratio is defined as a summary statistic of said collection of scores. Preferably, said sequences are obtained by low coverage sequencing.

Said normalization preferably occurs on the basis of a set of reference samples, whereby said reference samples are preferably, though not necessary, euploid or essentially euploid for the chromosome or chromosomal segment that corresponds to the target chromosome or chromosomal segment (i.e. the majority of the chromosomes or chromosomal segments in the reference samples that correspond to the target chromosome or chromosomal segment in the test sample are euploid). Such reference set have various sample sizes. A possible sample size can be e.g. 100 samples, such as 50 male and 50 female samples. It will be understood by a skilled person that the reference set can be freely chosen by the user.

By preference, said number of reads is recalibrated to correct for GC content and/or total number of reads obtained from said sample

By taking into account a set of scores derived of reads of chromosomes or chromosomal segments that include the target chromosome or chromosomal segment for calculation the collection of scores a more robust, sensitive and reliable parameter is obtained as compared to known prior art methods. Other than the known prior art methods, there is no need to make an assumption on the ploidy state of any of the chromosomes in the test sample. Even if multiple aneuploidies would be present in the test sample or a lot of technical or biological noise is present (e.g. coming from the presence of cancer or CNVs), the current parameter p still offers a valuable tool, whereas the methods known in the art may fail in those situations (Vandenberghe et al., "Non-invasive detection of genomic imbalances in Hodgkin/Reed-Sternberg cells in early and advanced stage Hodgkin's lymphoma by sequencing of circulating cell-free DNA: a technical proof-of-principle study", 2015). In fact, by defining a parameter according to the current invention, the parameter for the chromosome or region to be analyzed clearly stands out (i.e. is strongly increased/decreased), and does not disappear in the noise (i.e. only moderately or not increased/decreased). Moreover, for screening purpose, sensitivity is key, as it is important to have a reliable and trustworthy result, thereby minimizing the amount of false negatives. In fact, for screening purposes, it may be more important to have high sensitivity as compared to specificity.

The parameter according to the current invention allows robustly detecting and automatically classifying chromosomes, even in noisy data. By taking into account a collection of chromosomes or segments, including the target chromosome or segment i.e. the majority of information that is available in the dataset, most of the available information is used, coming to a more adequate assessment. For instance, if one would remove e.g. chromosome 1 (the largest chromosome, 7.9% of the genome), a large amount of data would be removed that would not be taken into account, thereby causing a distortion in the assessment.

In particular, the current invention is very useful in situations whereby a low number of reads or noisy data is obtained. The inventors found that in the latter situations, the parameter according to the current invention performed superior compared to other methodologies.

In a preferred embodiment, said scores are obtained on the basis of the genomic representation of the target chromosome or chromosomal segment (or a region thereof) and the genomic representation of all autosomes or other chromosomes, thereby including the target chromosome or chromosomal segment..

The parameter is compared to one or more cutoff values. The cutoff values may be determined from any number of suitable ways. Such ways include Bayesian-type likelihood method, sequential probability ratio testing (SPRT), false discovery, confidence interval, receiver operating characteristic (ROC). In a more preferred embodiment, said cutoff value is based on statistical consideration or is empirically determined by testing biological samples. The cutoff value can be validated by means of test data or a validation set and can, if necessary, be amended whenever more data is available.

Based on the comparison, a classification of whether a chromosomal aneuploidy exists for the target chromosome is determined. In one embodiment, the classification is a definitive yes or no. In another embodiment, a classification may be unclassifiable or uncertain. In yet another embodiment, the classification may be a score that is to be interpreted at a later date, for example, by a doctor. In another embodiment, the classification may occur on a genome-wide level. In yet another embodiment, the classification may be a score that determines the likelihood for the presence of genome-wide instability or the presence of a predefined CNV signature (i.e. a defined combination of CNVs or subchromosomal or chromosomal copy number aberrations).

In a further preferred method, secondary parameters from the read counts are calculated, which serve as an additional internal control for the usefulness of the parameter, the extend of the aneuploidy (if identified) and/or an indication for the reliability of the parameter, the biological sample or the sequences obtained thereof and thus the final assessment. Said secondary parameters can be a prerequisite of the presence of said aneuploidy and/or a measure of quality of the sample as well as a measure for genome-wide instability.

In one embodiment, such secondary parameter is calculated as the median of the Z-distribution of the read counts or a derivative thereof, for a target chromosome or a target chromosomal segment measured per bin or an aggregation of bins (i.e. windows). The latter secondary parameters allow assessing if the majority (more than 50%) of the windows in a chromosome is increased or decreased. The latter allows the detection of chromosomal and large subchromosomal aneuploidies. When less than 50% of the windows are affected, the secondary parameters will not be affected (e.g. for smaller CNVs).

In another embodiment, said secondary parameters may be calculated as the median of the absolute value of the Z-scores for the read counts or a derivative thereof, of the remaining chromosomes (that is a collection of chromosomes or segments that exclude the target chromosome or segment).

The latter secondary parameters allow the detection of e.g. the presence of technical or biological instabilities (cf. malignancies, cancer) and to discriminate these from maternal CNVs. If less than the windows of the other or all chromosomes are affected, these secondary parameters will not be affected. If more than 50% of the windows is affected, this will be derivable from said secondary parameters.

In another embodiment, the current invention also provides for a quality score (QS). QS allows to assess the overall variation across the genome. A low QS is an indication of a good sample processing and a low level of technical and biological noise. An increase in the QS can indicate two possible reasons. Either an error occurred during the sample processing. In general, the user will be requested to retrieve and test a new biological sample. This is typical for moderately increased QS scores. A strongly increased QS could be an indication of a highly aneuploid sample and the user will be encouraged to do a confirmatory test. Preferably, said QS is determined by calculating the standard deviations of all Z scores for chromosomes or chromosomal segments and optionally by removing the outliers thereof (i.e. the highest and lowest Z-scores in this collection).

In an embodiment of the current invention, the parameter p will be sufficient to discriminate between the presence and/or absence of an aneuploidy. In a more preferred embodiment of the current invention; both the parameter as the secondary parameters will be used to come to a decision with regard to the presence or absence of an aneuploidy. Preferably, also said secondary parameters will be compared to predefined threshold values.

In a preferred embodiment, said target chromosome or chromosomal segment comprise whole chromosomes amplifications and/or deletions of which are known to be associated with a cancer (e.g., as described herein). In certain embodiments said target chromosomes or chromosome segments comprise chromosome segment amplifications or deletions of which are known to be associated with one or more cancers. In certain embodiments the chromosome segments comprise substantially whole chromosome arms (e.g., as described herein). In certain embodiments the chromosome segments comprise whole chromosome aneuploidies. In certain embodiments the whole chromosome aneuploidies comprise a loss, while in certain other embodiments the whole chromosome aneuploidies comprise a gain (e.g., a gain or a loss as shown in Table 1). In certain embodiments the chromosome segments of interest are substantially arm-level segments comprising a p arm or a q arm of any one or more of chromosomes 1-22, X and Y. In certain embodiments the aneuploidies comprise an amplification of a substantial arm level segment of a chromosome or a deletion of a substantial arm level segment of a chromosome. In certain embodiments the chromosomal segments of interest substantially comprise one or more arms selected from the group consisting of 1q, 3q, 4p, 4q, 5p, 5q, 6p, 6q, 7p, 7q, 8p, 8q, 9p, 9q, 10p, 10q, 12p, 12q, 13q, 14q, 16p, 17p, 17q, 18p, 18q, 19p, 19q, 20p, 20q, 21q, and/or 22q. In certain embodiments the aneuploidies comprise an amplification of one or more arms selected from the group consisting of 1q, 3q, 4p, 4q, 5p, 5q, 6p, 6q, 7p, 7q, 8p, 8q, 9p, 9q, 10p, 10q, 12p, 12q, 13q, 14q, 16p, 17p, 17q, 18p, 18q, 19p, 19q, 20p, 20q, 21q, 22q. In certain embodiments the aneuploidies comprise a deletion of one or more arms selected from the group consisting of 1p, 3p, 4p, 4q, 5q, 6q, 8p, 8q, 9p, 9q, 10p, 10q, 11p, 11q, 13q, 14q, 15q, 16q, 17p, 17q, 18p, 18q, 19p, 19q, 22q. In certain embodiments the chromosomal segments of interest are segments that comprise a region and/or a gene shown in Table 3 and/or Table 5 and/or Table 4 and/or Table 6. In certain embodiments the aneuploidies comprise an amplification of a region and/or a gene shown in Table 3 and/or Table 5. In certain embodiments the aneuploidies comprise a deletion of a region and/or a gene shown in Table 4 and/or Table 6. In certain embodiments the chromosome segments of interest are segments known to contain one or more oncogenes and/or one or more tumor suppressor genes. In certain embodiments the aneuploidies comprise an amplification of one or more regions selected from the group consisting of 20Q13, 19q12, 1q21-1q23, 8p11-p12, and the ErbB2. In certain embodiments the aneuploidies comprise an amplification of one or more regions comprising a gene selected from the group consisting of MYC, ERBB2 (EGFR), CCND1 (Cyclin D1), FGFR1, FGFR2, HRAS, KRAS, MYB, MDM2, CCNE, NRAS, MET, ERBB1, CDK4, MYCB, ERBB2, AKT2, MDM2, BRAF, ARAF, CRAF, PIK3CA, AKT1, PTEN, STK11, MAP2K1, ALK, ROS1, CTNNB1, TP53, SMAD4, FBX7, FGFR3, NOTCH1, ERBB4 and CDK4, and the like. In certain embodiments the cancer is a cancer selected from the group consisting of leukemia, ALL, brain cancer, breast cancer, colorectal cancer, dedifferentiated liposarcoma, esophageal adenocarcinoma, esophageal squamous cell cancer, GIST, glioma, HCC, hepatocellular cancer, lung cancer, lung NSC, lung SC, medulloblastoma, melanoma, MPD, myeloproliferative disorder, cervical cancer, ovarian cancer, prostate cancer, and renal cancer.

In certain embodiments the biological sample comprise a sample selected from the group consisting of whole blood, a blood fraction, saliva/oral fluid, urine, a tissue biopsy, pleural fluid, pericardial fluid, cerebral spinal fluid, and peritoneal fluid.

In certain embodiments the detection of aneuploidies or genome-wide instability or CNV signatures or microsatellite instability (MSI) indicates a positive result and said method further comprises prescribing, initiating, and/or altering treatment of a human subject from whom the test sample was taken. In certain embodiments prescribing, initiating, and/or altering treatment of a human subject from whom the test sample was taken comprises prescribing and/or performing further diagnostics to determine the presence and/or severity of a cancer. In certain embodiments the further diagnostics comprise screening a sample from said subject for a biomarker of a cancer, and/or imaging said subject for a cancer. In certain embodiments when said method indicates the presence of neoplastic cells in said mammal, treating said mammal, or causing said mammal to be treated, to remove and/or to inhibit the growth or proliferation of said neoplastic cells. In certain embodiments treating the mammal comprises surgically removing the neoplastic (e.g., tumor) cells. In certain embodiments treating the mammal comprises performing radiotherapy or causing radiotherapy to be performed on said mammal to kill the neoplastic cells. In certain embodiments treating the mammal comprises administering or causing to be administered to said mammal anti-cancer drugs like Receptor Tyrosine Kinase (RTK) inhibitors, kinase inhibitors, CTLA4 inhibitors, PD1 inhibitors, PDL1 inhibitors, immunotherapy, tumor-targeting T-cell therapies, chimeric antigen receptor (CAR) T-cell therapy, cancer vaccines (e.g., matuzumab, erbitux, vectibix, nimotuzumab, matuzumab, panitumumab, fluorouracil, capecitabine, 5-trifluoromethyl-2'-deoxyuridine, methotrexate, raltitrexed, pemetrexed, cytosine arabinoside, 6-mercaptopurine, azathioprine, 6-thioguanine, pentostatin, fludarabine, cladribine, floxuridine, cyclophosphamide, neosar, ifosfamide, thiotepa, 1,3-bis(2-chloroethyl)-l-nitosourea, 1,-(2-chloroethyl)-3-cyclohexyl-initrosourea, hexamethylmelamine, busulfan, procarbazine, dacarbazine, chlorambucil, melphalan, cisplatin, carboplatin, oxaliplatin, bendamustine, carmustine, chloromethine, dacarbazine, fotemustine, lomustine, mannosulfan, nedaplatin, nimustine, prednimustine, ranimustine, satraplatin, semustine, streptozocin, temozolomide, treosulfan, triaziquone, triethylene melamine, thiotepa, triplatin tetranitrate, trofosfamide, uramustine, doxorubicin, daunorubicin, mitoxantrone, etoposide, topotecan, teniposide, irinotecan, camptosar, camptothecin, belotecan, rubitecan, vincristine, vinblastine, vinorelbine, vindesine, paclitaxel, docetaxel, abraxane, ixabepilone, larotaxel, ortataxel, tesetaxel, vinflunine, imatinib mesylate, sunitinib malate, sorafenib tosylate, nilotinib hydrochloride monohydrate/, tasigna, semaxanib, vandetanib, vatalanib, vemurafenib, dabrafenib, trametinib, ipilimumab, pembrolizumab, nivolumab, retinoic acid, a retinoic acid derivative, and the like).

Methods of monitoring a treatment of a subject for a cancer are also provided. In various embodiments the methods comprise performing a method for determining whether a subject has tumor-derived cell-free DNA in his or her peripheral blood, for confirming a cancer diagnosis, for aiding in the classification of a cancer, for assessing the treatment response, for monitoring the subject, for identifying the presence of a cancer and/or an increased risk of a cancer in a mammal as described herein on a sample from the subject or receiving the results of such a method performed on the sample before or during the treatment; and; performing the method again on a second sample from the subject or receiving the results of such a method performed on the second sample at a later time during or after the treatment; where a reduced number or severity of aneuploidy (e.g., a reduced aneuploidy frequency and/or a decrease or absence of certain aneuploidies) or a change in the CNV signature in the second measurement (e.g., as compared to the first measurement) can be an indicator of a positive course of treatment and the same or increased number or severity of aneuploidy or no or an adverse change in the CNV signature in the second measurement (e.g., as compared to the first measurement) can be an indicator of a negative course of treatment and, when said indicator is negative, adjusting said treatment regimen to a more aggressive treatment regimen and/or to a palliative treatment regimen.

**Table 1 Illustrative specific, recurrent chromosome gains and losses in human cancer (see, e.g., Gordon et al. (2012) Nature Rev. Genetics. 13: 189-203).**

| Chromosome | Gains (cancer type) | Losses (cancer type) |
|---|---|---|
| 1 | Multiple myeloma, Adenocarcinoma (breast) | Adenocarcinoma (kidney) |
| 2 | Hepatoblastoma, Ewing's sarcoma | |
| 3 | Multiple myeloma, Diffuse large B-cell lymphoma | Melanoma, Adenocarcinoma (kidney) |
| 4 | Acute lymphoblastic leukaemia | Adenocarcinoma (kidney) |
| 5 | Multiple myeloma, Adenocarcinoma (kidney) | |
| 6 | Acute lymphoblastic leukaemia, Wilms' tumour | Adenocarcinoma (kidney) |
| 7 | Adenocarcinoma (kidney) | Acute myeloid leukaemia Juvenile myelomonocytic leukaemia |
| 8 | Acute myeloid leukaemia, Chronic myeloid leukaemia, Ewing's sarcoma | Adenocarcinoma (kidney) |
| 9 | Multiple myeloma, Polycythaemia vera | |
| 10 | Acute lymphoblastic leukaemia, Adenocarcinoma (uterus) | Astrocytoma, Multiple myeloma |
| 11 | Multiple myeloma | |
| 12 | Chronic lymphocytic leukaemia, Wilms' tumor | Multiple myeloma |
| 13 | Acute myeloid leukaemia, Wilms tumor | Multiple myeloma |
| 14 | Acute lymphoblastic leukaemia | |

(continued)

| Chromosome | Gains (cancer type) | Losses (cancer type) |
|---|---|---|
| 15 | Acute lymphoblastic leukaemia | |
| 16 | Adenocarcinoma (kidney) | Multiple myeloma |
| 17 | Adenocarcinoma (kidney) Acute lymphoblastic leukaemia | |
| 18 | Acute lymphoblastic leukaemia, Wilms' tumour | Adenocarcinoma (kidney) |
| 19 | Multiple myeloma, Chronic myeloid leukaemia | Adenocarcinoma (Breast) Meningioma |
| 20 | Hepatoblastoma, Adenocarcinoma (kidney) | |
| 21 | Acute lymphoblastic leukaemia, Acute megakaryoblastic leukaemia | |
| X | Acute lymphoblastic leukaemia | |
| Y | Follicular lymphoma | |

In various embodiments, the method described herein can be used to detect and/or quantify whole chromosome aneuploidies that are associated with cancer generally, and/or that are associated with particular cancers. Thus, for example, in certain embodiments, detection and/or quantification of whole chromosome aneuploidies characterized by the gains or losses shown in Table 1 are contemplated.

Multiple studies have reported patterns of arm-level copy number variations across large numbers of cancer specimens (Lin et al. Cancer Res 68, 664-673 (2008); George et al. PLoS ONE 2, e255 (2007); Demichelis et al. Genes Chromosomes Cancer 48: 366-380 (2009); Beroukhim et al. Nature. 463(7283): 899-905 [2010]). It has additionally been observed that the frequency of arm-level copy number variations decreases with the length of chromosome arms. Adjusted for this trend, the majority of chromosome arms exhibit strong evidence of preferential gain or loss, but rarely both, across multiple cancer lineages (see, e.g., Beroukhim et al. Nature. 463(7283): 899-905 [2010]).

Accordingly, in one embodiment, methods described herein are used to determine arm level CNVs (CNVs comprising one chromosomal arm or substantially one chromosomal arm) in a sample. The CNVs can be determined in a test sample comprising a constitutional (germline) nucleic acid and the arm level CNVs can be identified in those constitutional nucleic acids. In certain embodiments arm level CNVs are identified (if present) in a sample comprising a mixture of nucleic acids (e.g., nucleic acids derived from normal and nucleic acids derived from neoplastic cells). In certain embodiments the sample is derived from a subject that is suspected or is known to have cancer e.g. carcinoma, sarcoma, lymphoma, leukemia, germ cell tumors, blastoma, and the like. In one embodiment, the sample is a plasma sample derived (processed) from peripheral blood that may comprise a mixture of cfDNA derived from normal and cancerous cells. In another embodiment, the biological sample that is used to determine whether a CNV is present is derived from a cells that, if a cancer is present, comprise a mixture of cancerous and non-cancerous cells from other biological tissues including, but not limited to biological fluids such as serum, sweat, tears, sputum, urine, sputum, ear flow, lymph, saliva, cerebrospinal fluid, ravages, bone marrow suspension, vaginal flow, transcervical lavage, brain fluid, ascites, milk, secretions of the respiratory, intestinal and genitourinary tracts, and leukophoresis samples, or in tissue biopsies, swabs, or smears. In other embodiments, the biological sample is a stool (fecal) sample.

In various embodiments the CNVs identified as indicative of the presence of a cancer or an increased risk for a cancer include, but are not limited to the arm level CNVs listed in Table 2. As illustrated in Table 2 certain CNVs that comprise a substantial arm-level gain are indicative of the presence of a cancer or an increased risk for a certain cancers. Thus, for example, a gain in 1q is indicative of the presence or increased risk for acute lymphoblastic leukemia (ALL), breast cancer, GIST, HCC, lung NSC, medulloblastoma, melanoma, MPD, ovarian cancer, and/or prostate cancer. A gain in 3q is indicative of the presence or increased risk for Esophageal Squamous cancer, Lung SC, and/or MPD. A gain in 7q is indicative of the presence or increased risk for colorectal cancer, glioma, HCC, lung NSC, medulloblastoma, melanoma, prostate cancer, and/or renal cancer. A gain in 7p is indicative of the presence or increased risk for breast cancer, colorectal cancer, esophageal adenocarcinoma, glioma, HCC, Lung NSC, medulloblastoma, melanoma, and/or renal cancer. A gain in 20q is indicative of the presence or increased risk for breast cancer, colorectal cancer, dedifferentiated liposarcoma, esophageal adenocarcinoma, esophageal squamous, glioma cancer, HCC, lung NSC, melanoma, ovarian cancer, and/or renal cancer, and so forth.

Similarly as illustrated in Table 2 certain CNVs that comprise a substantial arm-level loss are indicative of the presence of and/or an increased risk for certain cancers. Thus, for example, a loss in 1p is indicative of the presence or increased risk for gastrointestinal stromal tumor. A loss in 4q is indicative of the presence or increased risk for colorectal cancer, esophageal adenocarcinoma, lung sc, melanoma, ovarian cancer, and/or renal cancer. A loss in 17p is indicative of the presence or increased risk for breast cancer, colorectal cancer, esophageal adenocarcinoma, HCC, lung NSC, lung SC, and/or ovarian cancer, and the like.

**Table 2 Significant arm-level chromosomal segment copy number alterations in each of 16 cancer subtypes**

| Arm | | Cancer Types Significantly Gained In | Cancer Types Significantly Lost In | Oncogene/Tumor Suppressor Gene |
|---|---|---|---|---|
| | 1p | - | GIST | |
| | 1q | ALL, Breast, GIST, HCC, Lung NSC, Medulloblastoma, Melanoma, MPD, Ovarian, Prostate | - | |
| | 3p | - | Esophageal Squamous, Lung NSC, Lung SC, Renal | VHL |
| | 3q | Esophageal Squamous, Lung SC, MPD | - | |
| | 4p | ALL | Breast, Esophageal Adenocarcinoma, Renal | |
| | 4q | ALL | Colorectal, Esophageal Adenocarcinoma, Lung SC, Melanoma, Ovarian, Renal | |
| | 5p | Esophageal Squamous, HCC, Lung NSC, Lung SC, Renal | - | TERT |
| | 5q | HCC, Renal | Esophageal Adenocarcinoma, Lung NSC | APC |
| | 6p | ALL, HCC, Lung NSC, Melanoma | - | |
| | 6q | ALL | Melanoma, Renal | |
| | 7p | Breast, Colorectal, Esophageal Adenocarcinoma, Glioma, HCC, Lung NSC, Medulloblastoma, Melanoma, Renal | - | EGFR |
| | 7q | Colorectal, Glioma, HCC, Lung NSC, Medulloblastoma, Melanoma, Prostate, Renal | - | BRAF, MET |
| | 8p | ALL, MPD | Breast, HCC, Lung NSC, Medulloblastoma, Prostate, Renal | |
| | 8q | ALL, Breast, Colorectal, | Medulloblastoma | MYC |

(continued)

| | Cancer Types | Cancer Types | Oncogene/Tumor |
|---|---|---|---|
| | Esophageal Adenocarcinoma, Esophageal Squamous, HCC, Lung NSC, MPD, Ovarian, Prostate | | |
| 9p | MPD | ALL, Breast, Esophageal Adenocarcinoma, Lung NSC, Melanoma, Ovarian, Renal | CDKN2A/B |
| 9q | ALL, MPD | Lung NSC, Melanoma, Ovarian, Renal | |
| 10p | ALL | Glioma, Lung SC, Melanoma | |
| 10q | ALL | Glioma, Lung SC, Medulloblastoma, Melanoma | PTEN |
| 11p | - | Medulloblastoma | WT1 |
| 11q | - | Dedifferentiated Liposarcoma, Medulloblastoma, Melanoma | ATM |
| 12p | Colorectal, Renal | - | KRAS |
| 12q | Renal | - | |
| 13q | Colorectal | Breast, Dedifferentiated Liposarcoma, Glioma, Lung NSC, Ovarian | RB1/BRCA2 |
| 14q | ALL, Lung NSC, Lung SC, Prostate | GIST, Melanoma, Renal | |
| 15q | - | GIST, Lung NSC, Lung SC, Ovarian | |
| 16p | Breast | - | |
| 16q | - | Breast, HCC, Medulloblastoma, Ovarian, Prostate | |
| 17p | ALL | Breast, Colorectal, Esophageal Adenocarcinoma, HCC, Lung NSC, Lung SC, Ovarian | TP53 |
| 17q | ALL, HCC, Lung NSC, Medulloblastoma | Breast, Ovarian | ERBB2, NF1/BRCA1 |
| 18p | ALL, Medulloblastoma | Colorectal, Lung NSC | |
| 18q | ALL, Medulloblastoma | Colorectal, Esophageal Adenocarcinoma, Lung NSC | SMAD2, SMAD4 |
| 19p | Glioma | Esophageal Adenocarcinoma, | |

(continued)

| | | Cancer Types | Cancer Types | Oncogene/Tumor |
|---|---|---|---|---|
| | | | Lung NSC, Melanoma, | |
| | | | Ovarian | |
| | 19q | Glioma, Lung SC | Esophageal Adenocarcinoma, | |
| | | | Lung NSC | |
| | 20p | Breast, Colorectal, Esophageal | - | |
| | | Adenocarcinoma, Esophageal | | |
| | | Squamous, GIST, Glioma, HCC, | | |
| | | Lung NSC, Melanoma, Renal | | |
| | 20q | Breast, Colorectal, | - | |
| | | Dedifferentiated Liposarcoma, | | |
| | | Esophageal Adenocarcinoma, | | |
| | | Esophageal Squamous, Glioma, | | |
| | | HCC, Lung NSC, Melanoma, | | |
| | | Ovarian, Renal | | |
| | 21q | ALL, GIST, MPD | - | |
| | 22q | Melanoma | Breast, Colorectal, | NF2 |
| | | | Ded ifferentiated Liposarcoma, | |
| | | | Esophageal Adenocarcinoma, | |
| | | | GIST, Lung NSC, Lung SC, | |
| | | | Ovarian, Prostate | |

The examples of associations between arm level copy number variations are intended to be illustrative and not limiting. Other arm level copy number variations and their cancer associations are known to those of skill in the art.

Other copy number variations that do not span a significant portion of a chromosome or chromosome arm, such as CNVs of 1 kb to 1Mb, or 1 kb to 10 Mb, or 100 kb to 10 Mb, or 1 kb to 50 Mb, or 2 bp to 10 Mb or 2 bp to 50 Mb could equally be informative for the detection or confirmation of the presence of tumor-derived cell-free DNA.

As indicated above, in certain embodiment, the method described herein can be used to determine the presence or absence of a chromosomal amplification. In some embodiments, the chromosomal amplification is the gain of one or more entire chromosomes. In other embodiments, the chromosomal amplification is the gain of one or more segments of a chromosome. In yet other embodiments, the chromosomal amplification is the gain of two or more segments of two or more chromosomes. In various embodiments, the chromosomal amplification can involve the gain of one or more oncogenes.

Dominantly acting genes associated with human solid tumors typically exert their effect by overexpression or altered expression. Gene amplification is a common mechanism leading to upregulation of gene expression. Evidence from cytogenetic studies indicates that significant amplification occurs in over 50% of human breast cancers. Most notably, the amplification of the proto-oncogene human epidermal growth factor receptor 2 (HER2) located on chromosome 17 (17(17q21-q22)), results in overexpression of HER2 receptors on the cell surface leading to excessive and dysregulated signaling in breast cancer and other malignancies (Park et al., Clinical Breast Cancer 8:392-401 [2008]). A variety of oncogenes have been found to be amplified in other human malignancies. Examples of the amplification of cellular oncogenes in human tumors include amplifications of: c-myc in promyelocytic leukemia cell line HL60, and in small-cell lung carcinoma cell lines, N-myc in primary neuroblastomas (stages III and IV), neuroblastoma cell lines, retinoblastoma cell line and primary tumors, and small-cell lung carcinoma lines and tumors, L-myc in small-cell lung carcinoma cell lines and tumors, c-myb in acute myeloid leukemia and in colon carcinoma cell lines, c-erbb in epidermoid carcinoma cell, and primary gliomas, c-K-ras-2 or KRAS in primary carcinomas of lung, colon, bladder, and rectum, N-ras or NRAS in mammary carcinoma cell line (Varmus H., Ann Rev Genetics 18: 553-612 (1984) [cited in Watson et al.,

Molecular Biology of the Gene (4th ed.; Benjamin/Cummings Publishing Co. 1987)].

Amplifications of oncogenes are a common cause of many types of cancer, as is the case with P70-S6 Kinase 1 amplification and breast cancer. In such cases the genetic amplification occurs in a somatic cell and affects only the genome of the cancer cells themselves, not the entire organism, much less any subsequent offspring. Other examples of oncogenes that are amplified in human cancers include MYC, ERBB2 (EFGR), CCND1 (Cyclin D1), FGFR1 and FGFR2 in breast cancer, MYC and ERBB2 in cervical cancer, HRAS, KRAS, NRAS, and MYB in colorectal cancer, MYC, CCND1 and MDM2 in esophageal cancer, CCNE, KRAS and MET in gastric cancer, ERBB1, and CDK4 in glioblastoma, CCND1, ERBB1, and MYC in head and neck cancer, CCND1 in hepatocellular cancer, MYCB in neuroblastoma, MYC, ERBB2 and AKT2 in ovarian cancer, MDM2 and CDK4 in sarcoma, NRAS in melanoma and MYC in small cell lung cancer. In one embodiment, the present method can be used to determine the presence or absence of amplification of an oncogene associated with a cancer. In some embodiments, the amplified oncogene is associated with breast cancer, cervical cancer, colorectal cancer, esophageal cancer, gastric cancer, glioblastoma, head and neck cancer, hepatocellular cancer, neuroblastoma, ovarian cancer, melanoma, prostate cancer, sarcoma, and small cell lung cancer.

In one embodiment, the present method can be used to determine the presence or absence of a chromosomal deletion. In some embodiments, the chromosomal deletion is the loss of one or more entire chromosomes. In other embodiments, the chromosomal deletion is the loss of one or more segments of a chromosome. In yet other embodiments, the chromosomal deletion is the loss of two or more segments of two or more chromosomes. The chromosomal deletion can involve the loss of one or more tumor suppressor genes.

Chromosomal deletions involving tumor suppressor genes are believed to play an important role in the development and progression of solid tumors. The retinoblastoma tumor suppressor gene (Rb-1), located in chromosome 13q14, is the most extensively characterized tumor suppressor gene. The Rb-1 gene product, a 105 kDa nuclear phosphoprotein, apparently plays an important role in cell cycle regulation (Howe et al., Proc Natl Acad Sci (USA) 87:5883-5887 [1990]). Altered or lost expression of the Rb protein is caused by inactivation of both gene alleles either through a point mutation or a chromosomal deletion. Rb-i gene alterations have been found to be present not only in retinoblastomas but also in other malignancies such as osteosarcomas, small cell lung cancer (Rygaard et al., Cancer Res 50: 5312-5317 [1990)]) and breast cancer. Restriction fragment length polymorphism (RFLP) studies have indicated that such tumor types have frequently lost heterozygosity at 13q suggesting that one of the Rb-1 gene alleles has been lost due to a gross chromosomal deletion (Bowcock et al., Am J Hum Genet, 46: 12 [1990]). Chromosome 1 abnormalities including duplications, deletions and unbalanced translocations involving chromosome 6 and other partner chromosomes indicate that regions of chromosome 1, in particular 1q21-1q32 and 1p11-13, might harbor oncogenes or tumor suppressor genes that are pathogenetically relevant to both chronic and advanced phases of myeloproliferative neoplasms (Caramazza et al., Eur J Hematol 84:191-200 [2010]). Myeloproliferative neoplasms are also associated with deletions of chromosome 5. Complete loss or interstitial deletions of chromosome 5 are the most common karyotypic abnormality in myelodysplastic syndromes (MDSs). Isolated del(5q)/5q-MDS patients have a more favorable prognosis than those with additional karyotypic defects, who tend to develop myeloproliferative neoplasms (MPNs) and acute myeloid leukemia. The frequency of unbalanced chromosome 5 deletions has led to the idea that 5q harbors one or more tumor-suppressor genes that have fundamental roles in the growth control of hematopoietic stem/progenitor cells (HSCs/HPCs). Cytogenetic mapping of commonly deleted regions (CDRs) centered on 5q31 and 5q32 identified candidate tumor-suppressor genes, including the ribosomal subunit RPS14, the transcription factor Egrl/Krox20 and the cytoskeletal remodeling protein, alpha-catenin (Eisenmann et al., Oncogene 28:3429-3441 [2009]). Cytogenetic and allelotyping studies of fresh tumors and tumor cell lines have shown that allelic loss from several distinct regions on chromosome 3p, including 3p25, 3p21-22, 3p21.3, 3p12-13 and 3p14, are the earliest and most frequent genomic abnormalities involved in a wide spectrum of major epithelial cancers of lung, breast, kidney, head and neck, ovary, cervix, colon, pancreas, esophagus, bladder and other organs. Several tumor suppressor genes have been mapped to the chromosome 3p region, and are thought that interstitial deletions or promoter hypermethylation precede the loss of the 3p or the entire chromosome 3 in the development of carcinomas (Angeloni D., Briefings Functional Genomics 6:19-39 [2007]).

Newborns and children with Down syndrome (DS) often present with congenital transient leukemia and have an increased risk of acute myeloid leukemia and acute lymphoblastic leukemia. Chromosome 21, harboring about 300 genes, may be involved in numerous structural aberrations, e.g., translocations, deletions, and amplifications, in leukemias, lymphomas, and solid tumors. Moreover, genes located on chromosome 21 have been identified that play an important role in tumorigenesis. Somatic numerical as well as structural chromosome 21 aberrations are associated with leukemias, and specific genes including RUNX1, TMPRSS2, and TFF, which are located in 21q, play a role in tumorigenesis (Fonatsch C Gene Chromosomes Cancer 49:497-508 [2010]).

In view of the foregoing, in various embodiments the method described herein can be used to determine the segment CNVs that are known to comprise one or more oncogenes or tumor suppressor genes, and/or that are known to be associated with a cancer or an increased risk of cancer. In certain embodiments, the CNVs can be determined in a test sample comprising a constitutional (germline) nucleic acid and the segment can be identified in those constitutional nucleic acids. In certain embodiments segment CNVs are identified (if present) in a sample comprising a mixture of nucleic acids (e.g., nucleic acids derived from normal and nucleic acids derived from neoplastic cells). In certain embodiments the sample is derived from a subject that is suspected or is known to have cancer e.g. carcinoma, sarcoma, lymphoma, leukemia, germ cell tumors, blastoma, and the like. In one embodiment, the sample is a plasma sample derived (processed) from peripheral blood that may comprise a mixture of cfDNA derived from normal and cancerous cells. In another embodiment, the biological sample that is used to determine whether a CNV is present is derived from a cells that, if a cancer is present, comprises a mixture of cancerous and non-cancerous cells from other biological tissues including, but not limited to biological fluids such as serum, sweat, tears, sputum, urine, sputum, ear flow, lymph, saliva, cerebrospinal fluid, ravages, bone marrow suspension, vaginal flow, transcervical lavage, brain fluid, ascites, milk, secretions of the respiratory, intestinal and genitourinary tracts, and leukophoresis samples, or in tissue biopsies, swabs, or smears. In other embodiments, the biological sample is a stool (fecal) sample.

The CNVs used to determine presence of a cancer and/or increased risk for a cancer can comprise amplification or deletions.

In various embodiments the CNVs identified as indicative of the presence of a cancer or an increased risk for a cancer include one or more of the amplifications shown in Table 3.

**Table 3 Illustrative, but non-limiting chromosomal segments characterized by amplifications that are ass ociated with cancers.**

| Peak region | Length(Mb) | Cancer types identified in this analysis but notprior publications |
|---|---|---|
| | | |
| chr1: 119996566-120303234 | 0.228 | Breast, Lung SC, Melanoma |
| chr1: 148661965-149063439 | 0.35 | Breast, Dedifferentiated liposarcoma, Esophageal adenocarcinoma, Hepatocellular, Lung SC, Melanoma, Ovarian, Prostate, Renal |
| chr1: 1-5160566 | 4.416 | Esophageal adenocarcinoma, Ovarian |
| chr1: 158317017-159953843 | 1.627 | Dedifferentiated liposarcoma, Esophageal adenocarcinoma, Prostate, Renal |
| chr1: 169549478-170484405 | 0.889 | Colorectal, Dedifferentiated liposarcoma, Prostate, Renal |
| chr1: 201678483-203358272 | 1.471 | Prostate |
| chr1: 241364021-1247249719 | 5.678 | Lung NSC, Melanoma, Ovarian |
| chr1: 39907605-40263248 | 0.319 | Acute lymphoblastic leukemia, Breast, Lung NSC, Lung SC |
| chr1: 58658784-60221344 | 1.544 | Breast, Dedifferentiated liposarcoma, Lung SC |
| chr3: 170024984-173604597 | 3.496 | Breast, Esophageal adenocarcinoma, Glioma |
| chr3: 178149984-199501827 | 21.123 | Esophageal squamous, Lung NSC |
| chr3: 86250885- | 8.795 | Lung SC, Melanoma |

(continued)

| Peak region | Length(Mb) | Cancer types identified in this analysis but notprior publications |
|---|---|---|
| 95164178 | | |
| chr4: 54471680-55980061 | 1.449 | Lung NSC |
| chr5: 1212750-1378766 | 0.115 | Dedifferentiated liposarcoma |
| chr5: 174477192-180857866 | 6.124 | Breast, Lung NSC |
| chr5: 45312870-49697231 | 4.206 | Lung SC |
| chr6: 1-23628840 | 23.516 | Esophageal adenocarcinoma |
| chr6: 135561194-135665525 | 0.092 | Breast, Esophageal adenocarcinoma |
| chr6: 43556800-44361368 | 0.72 | Esophageal adenocarcinoma, Hepatocellular, Ovarian |
| chr6: 63255006-65243766 | 1.988 | Esophageal adenocarcinoma, Lung NSC |
| chr7: 115981465-116676953 | 0.69 | Esophageal adenocarcinoma, Lung NSC, Melanoma, Ovarian |
| chr7: 54899301-55275419 | 0.363 | Esophageal adenocarcinoma, Esophageal squamous |
| chr7: 89924533-98997268 | 9.068 | Breast, Esophageal adenocarcinoma, Esophageal squamous, Ovarian |
| chr8: 101163387-103693879 | 2.516 | Lung NSC, Melanoma, Ovarian |
| chr8: 116186189-120600761 | 4.4 | Breast, Hepatocellular, Lung NSC, Ovarian |
| chr8: 128774432-128849112 | 0.009 | Esophageal adenocarcinoma, Esophageal squamous, Hepatocellular, Lung SC, Medulloblastoma, Myeloproliferative disorder, Ovarian |
| chr8: 140458177-146274826 | 5.784 | Lung NSC, Medulloblastoma, Melanoma, Ovarian |
| chr8: 38252951-38460772 | 0.167 | Colorectal, Esophageal adenocarcinoma, Esophageal squamous |
| chr8: 42006632-42404492 | 0.257 | Esophageal adenocarcinoma, Lung NSC, Lung SC, Ovarian, Prostate |
| chr8: 81242335- | 0.717 | Breast, Melanoma |

(continued)

| Peak region | Length(Mb) | Cancer types identified in this analysis but notprior publications |
|---|---|---|
| 81979194 | | |
| chr9: 137859478-140273252 | 2.29 | Colorectal, Dedifferentiated liposarcoma |
| chr10: 74560456-82020637 | 7.455 | Breast, Ovarian, Prostate |
| chr11: 101433436-102134907 | 0.683 | Lung NSC, Lung SC |
| chr11: 32027116-37799354 | 5.744 | Breast, Dedifferentiated liposarcoma, Lung NSC, Lung SC |
| chr11: 69098089-69278404 | 0.161 | Dedifferentiated liposarcoma, Esophageal adenocarcinoma, Hepatocellular, Lung SC, Ovarian |
| chr11: 76699529-78005085 | 1.286 | Dedifferentiated liposarcoma, Esophageal adenocarcinoma, Lung SC, Ovarian |
| chr12: 1-1311104 | 1.271 | Lung NSC |
| chr12: 25189655-25352305 | 0.112 | Acute lymphoblastic leukemia, Esophageal adenocarcinoma, Esophageal squamous, Ovarian |
| chr12: 30999223-32594050 | 1.577 | Acute lymphoblastic leukemia, Colorectal, Esophageal adenocarcinoma, Esophageal squamous, Lung NSC, Lung SC |
| chr12: 38788913-42596599 | 3.779 | Breast, Colorectal, Dedifferentiated liposarcoma, Esophageal squamous, Lung NSC, Lung SC |
| chr12: 56419524-56488685 | 0.021 | Dedifferentiated liposarcoma, Melanoma, Renal |
| chr12: 64461446-64607139 | 0.041 | Dedifferentiated liposarcoma, Renal |
| chr12: 66458200-66543552 | 0.058 | Dedifferentiated liposarcoma, Esophageal squamous, Renal |
| chr12: 67440273-67566002 | 0.067 | Breast, Dedifferentiated liposarcoma, Esophageal squamous, Melanoma, Renal |
| chr12: 68249634-68327233 | 0.06 | Breast, Dedifferentiated liposarcoma, Esophageal squamous, Renal |
| chr12: 70849987-70966467 | 0.036 | Dedifferentiated liposarcoma, Renal |

(continued)

| Peak region | Length(Mb) | Cancer types identified in this analysis but notprior publications |
|---|---|---|
| chr12: 72596017-73080626 | 0.23 | Renal |
| chr12: 76852527-77064746 | 0.158 | Dedifferentiated liposarcoma |
| chr12: 85072329-85674601 | 0.272 | Dedifferentiated liposarcoma |
| chr12: 95089777-95350380 | 0.161 | Dedifferentiated liposarcoma |
| chr13: 108477140-110084607 | 1.6 | Breast, Esophageal adenocarcinoma, Lung NSC, Lung SC |
| chr13: 1-40829685 | 22.732 | Acute lymphoblastic leukemia, Esophageal adenocarcinoma |
| chr13: 89500014-93206506 | 3.597 | Breast, Esophageal adenocarcinoma, Medulloblastoma |
| chr14: 106074644-106368585 | 0.203 | Esophageal squamous |
| chr14: 1-23145193 | 3.635 | Acute lymphoblastic leukemia, Esophageal squamous, Hepatocellular, Lung SC |
| chr14: 35708407-36097605 | 0.383 | Breast, Esophageal adenocarcinoma, Esophageal squamous, Hepatocellular, Prostate |
| chr15: 96891354-97698742 | 0.778 | Breast, Colorectal, Esophageal adenocarcinoma, Lung NSC, Medulloblastoma, Melanoma |
| chr17: 18837023-19933105 | 0.815 | Breast, Hepatocellular |
| chr17: 22479313-22877776 | 0.382 | Breast, Lung NSC |
| chr17: 24112056-24310787 | 0.114 | Breast, Lung NSC |
| chr17: 35067383-35272328 | 0.149 | Colorectal, Esophageal adenocarcinoma, Esophageal squamous |
| chr17: 44673157-45060263 | 0.351 | Melanoma |
| chr17: 55144989-55540417 | 0.31 | Lung NSC, Medulloblastoma, Melanoma, Ovarian |

(continued)

| Peak region | Length(Mb) | Cancer types identified in this analysis but notprior publications |
|---|---|---|
| chr17: 62318152-63890591 | 1.519 | Breast, Lung NSC, Melanoma, Ovarian |
| chr17: 70767943-71305641 | 0.537 | Breast, Lung NSC, Melanoma, Ovarian |
| chr18: 17749667-22797232 | 5.029 | Colorectal, Esophageal adenocarcinoma, Ovarian |
| chr19: 34975531-35098303 | 0.096 | Breast, Esophageal adenocarcinoma, Esophageal squamous |
| chr19: 43177306-45393020 | 2.17 | Lung NSC, Ovarian |
| chr19: 59066340-59471027 | 0.321 | Breast, Lung NSC, Ovarian |
| chr2: 15977811-16073001 | 0.056 | Lung SC |
| chr20: 29526118-29834552 | 0.246 | Ovarian |
| chr20: 51603033-51989829 | 0.371 | Hepatocellular, Lung NSC, Ovarian |
| chr20: 61329497-62435964 | 0.935 | Hepatocellular, Lung NSC |
| chr22: 19172385-19746441 | 0.487 | Colorectal, Melanoma, Ovarian |
| chrX: 152729030-154913754 | 1.748 | Breast, Lung NSC, Renal |
| chrX: 66436234- | 0.267 | Ovarian, Prostate |

In certain embodiments in combination with the amplifications described above (herein), or separately, the CNVs identified as indicative of the presence of a cancer or an increased risk for a cancer include one or more of the deletions shown in Table 4

**Table 3 Illustrative but not limiting chromsomal segments characterized by deletions that are associated with cancers.**

| Peak region | Length (Mb) | Cancer types identified in this analysis but not prior publications |
|---|---|---|
| | | |
| chr1: 110339388-119426489 | 1p13.2 | Acute lymphoblastic leukemia, |
| | | Esophageal adenocarcinoma, Lung NSC, |
| | | Lung SC, Melanoma, Ovarian, Prostate |

(continued)

| Peak region | Length (Mb) | Cancer types identified in this analysis but not prior publications |
|---|---|---|
| chr1: 223876038-247249719 | 1q43 | Acute lymphoblastic leukemia, Breast, |
| | | Lung SC, Melanoma, Prostate |
| chr1: 26377344-27532551 | 1p36.11 | Breast, Esophageal adenocarcinoma, |
| | | Esophageal squamous, Lung NSC, Lung |
| | | SC, Medulloblastoma, Myeloproliferative |
| | | disorder, Ovarian, Prostate |
| chr1: 3756302-6867390 | 1p36.31 | Acute lymphoblastic leukemia, Breast, |
| | | Esophageal squamous, Hepatocellular, |
| | | Lung NSC, Lung SC, Medulloblastoma, |
| | | Myeloproliferative disorder, Ovarian, |
| | | Prostate, Renal |
| chr1: 71284749-74440273 | 11p31.1 | Breast, Esophageal adenocarcinoma, |
| | | Glioma, Hepatocellular, Lung NSC, Lung |
| | | SC, Melanoma, Ovarian, Renal |
| chr2: 1-15244284 | 2p25.3 | Lung NSC, Ovarian |
| chr2: 138479322-143365272 | 2q22.1 | Breast, Colorectal, Esophageal |
| | | adenocarcinoma, Esophageal squamous, |
| | | Hepatocellular, Lung NSC, Ovarian, |
| | | Prostate, Renal |
| chr2: 204533830-206266883 | 2q33.2 | Esophageal adenocarcinoma, |
| | | Hepatocellular, Lung NSC, |
| | | Medulloblastoma, Renal |
| chr2: 241477619-242951149 | 2q37.3 | Breast, Dedifferentiated liposarcoma, |
| | | Esophageal adenocarcinoma, Esophageal |
| | | squamous, Hepatocellular, Lung NSC, |
| | | Lung SC, Medulloblastoma, Melanoma, |
| | | Ovarian, Renal |
| chr3: 116900556-120107320 | 3q13.31 | Dedifferentiated liposarcoma, |
| | | Esophageal adenocarcinoma, Hepatocellular, |
| | | Lung NSC, Melanoma, Myeloproliferative |
| | | disorder, Prostate |
| chr3: 1-2121282 | 3p26.3 | Colorectal, Dedifferentiated liposarcoma, |
| | | Esophageal adenocarcinoma, Lung NSC, |
| | | Melanoma, Myeloproliferative disorder |
| chr3: 175446835-178263192 | 3q26.31 | Acute lymphoblastic leukemia, |
| | | Dedifferentiated liposarcoma, Esophageal |
| | | adenocarcinoma, Lung NSC, Melanoma, |

(continued)

| Peak region | Length (Mb) | Cancer types identified in this analysis but not prior publications |
|---|---|---|
| | | Myeloproliferative disorder, Prostate |
| chr3: 58626894-61524607 | 3p14.2 | Breast, Colorectal, Dedifferentiated |
| | | liposarcoma, Esophageal adenocarcinoma, |
| | | Esophageal squamous, Hepatocellular, |
| | | Lung NSC, Lung SC, Medulloblastoma, |
| | | Melanoma, Myeloproliferative disorder, |
| | | Ovarian, Prostate, Renal |
| chr4: 1-435793 | 4p16.3 | Myeloproliferative disorder |
| chr4: 186684565-191273063 | 4q35.2 | Breast, Esophageal adenocarcinoma, |
| | | Esophageal squamous, Lung NSC, |
| | | Medulloblastoma, Melanoma, Prostate, |
| | | Renal |
| chr4: 91089383-93486891 | 4q22.1 | Acute lymphoblastic leukemia, Esophageal |
| | | adenocarcinoma, Hepatocellular, Lung |
| | | NSC, Renal |
| chr5: 177541057-180857866 | 5q35.3 | Breast, Lung NSC, Myeloproliferative |
| | | disorder, Ovarian |
| chr5: 57754754-59053198 | 5q11.2 | Breast, Colorectal, Dedifferentiated |
| | | liposarcoma, Esophageal adenocarcinoma, |
| | | Esophageal squamous, Lung SC, |
| | | Melanoma, Myeloproliferative disorder, |
| | | Ovarian, Prostate |
| chr5: 85837489-133480433 | 5q21.1 | Colorectal, Dedifferentiated liposarcoma, |
| | | Lung NSC, Lung SC, Myeloproliferative |
| | | disorder, Ovarian |
| chr6: 101000242-121511318 | 6q22.1 | Colorectal, Lung NSC, Lung SC |
| | | |
| chr6: 1543157-2570302 | 6p25.3 | Colorectal, Dedifferentiated liposarcoma, |
| | | Esophageal adenocarcinoma, Lung NSC, |
| | | Lung SC, Ovarian, Prostate |
| chr6: 161612277-163134099 | 6q26 | Colorectal, Esophageal adenocarcinoma, |
| | | Esophageal squamous, Lung NSC, Lung |
| | | SC, Ovarian, Prostate |
| chr6: 76630464-105342994 | 6q16.1 | Colorectal, Hepatocellular, Lung NSC |
| | | |
| chr7: 141592807-142264966 | 7q34 | Breast, Colorectal, Esophageal |
| | | adenocarcinoma, Esophageal squamous, |

(continued)

| Peak region | Length (Mb) | Cancer types identified in this analysis but not prior publications |
|---|---|---|
| | | Hepatocellular, Lung NSC, Ovarian, |
| | | Prostate, Renal |
| chr7: 144118814-148066271 | 7q35 | Breast, Esophageal adenocarcinoma, |
| | | Esophageal squamous, Lung NSC, |
| | | Melanoma, Myeloproliferative disorder, |
| | | Ovarian |
| chr7: 156893473-158821424 | q36.3 | Breast, Esophageal adenocarcinoma, |
| | | Esophageal squamous, Lung NSC, |
| | | Melanoma, Myeloproliferative disorder, |
| | | Ovarian, Prostate |
| chr7: 3046420-4279470 | 7p22.2 | Melanoma, Myeloproliferative disorder, |
| | | Ovarian |
| chr7: 65877239-79629882 | 7q21.11 | Breast, Medulloblastoma, Melanoma, |
| | | Myeloproliferative disorder, Ovarian |
| chr8: 1-392555 | 8p23.3 | Acute lymphoblastic leukemia, Breast, |
| | | Myeloproliferative disorder |
| chr8: 2053441-6259545 | 8p23.2 | Acute lymphoblastic leukemia, |
| | | Dedifferentiated liposarcoma, Esophageal |
| | | adenocarcinoma, Esophageal squamous, |
| | | Hepatocellular, Lung NSC, |
| | | Myeloproliferative disorder |
| chr8: 22125332-30139123 | 8p21.2 | Acute lymphoblastic leukemia, |
| | | Dedifferentiated liposarcoma, |
| | | Hepatocellular, Myeloproliferative |
| | | disorder, Ovarian, Renal |
| chr8: 39008109-41238710 | 8p11.22 | Acute lymphoblastic leukemia, Breast, |
| | | Dedifferentiated liposarcoma, Esophageal |
| | | squamous, Hepatocellular, Lung NSC, |
| | | Myeloproliferative disorder, Renal |
| chr8: 42971602-72924037 | 8q11.22 | Breast, Dedifferentiated liposarcoma, |
| | | Esophageal squamous, Hepatocellular, |
| | | Lung NSC, Myeloproliferative disorder, |
| | | Renal |
| chr9: 1-708871 | 9p24.3 | Acute lymphoblastic leukemia, Breast, |
| | | Lung NSC, Myeloproliferative disorder, |
| | | Ovarian, Prostate |

(continued)

| Peak region | Length (Mb) | Cancer types identified in this analysis but not prior publications |
|---|---|---|
| chr9: 21489625-22474701 | 9p21.3 | Colorectal, Esophageal adenocarcinoma, |
| | | Esophageal squamous, Myeloproliferative |
| | | disorder, Ovarian |
| chr9: 36365710-37139941 | 9p13.2 | Myeloproliferative disorder |
| | | |
| chr9: 7161607-12713130 | 9p24.1 | Acute lymphoblastic leukemia, Breast, |
| | | Colorectal, Esophageal adenocarcinoma, |
| | | Hepatocellular, Lung SC, |
| | | Medulloblastoma, Melanoma, |
| | | Myeloproliferative disorder, Ovarian, |
| | | Prostate, Renal |
| chr10: 1-1042949 | 10p15.3 | Colorectal, Lung NSC, Lung SC, Ovarian, |
| | | Prostate, Renal |
| chr10: 129812260-135374737 | 10q26.3 | Breast, Colorectal, Glioma, Lung NSC, |
| | | Lung SC, Melanoma, Ovarian, Renal |
| chr10: 52313829-53768264 | 10q11.23 | Colorectal, Lung NSC, Lung SC, Ovarian, |
| | | Renal |
| chr10: 89467202-90419015 | 10q23.31 | Breast, Lung SC, Ovarian, Renal |
| | | |
| chr11: 107086196-116175885 | 11q23.1 | Esophageal adenocarcinoma, |
| | | Medulloblastoma, Renal |
| chr11: 1-1391954 | 11p15.5 | Breast, Dedifferentiated liposarcoma, |
| | | Esophageal adenocarcinoma, Lung NSC, |
| | | Medulloblastoma, Ovarian |
| chr11: 130280899-134452384 | 11q25 | Esophageal adenocarcinoma, Esophageal |
| | | squamous, Hepatocellular, Lung NSC, |
| | | Medulloblastoma, Renal |
| chr11: 82612034-85091467 | 11q14.1 | Melanoma, Renal |
| | | |
| chr12: 11410696-12118386 | 12p13.2 | Breast, Hepatocellular, Myeloproliferative |
| | | disorder, Prostate |
| chr12: 131913408-132349534 | 12q24.33 | Dedifferentiated liposarcoma, Lung NSC, |
| | | Myeloproliferative disorder |
| chr12: 97551177-99047626 | 12q23.1 | Breast, Colorectal, Esophageal squamous, |
| | | Lung NSC, Myeloproliferative disorder |
| chr13: 111767404-114142980 | 13q34 | Breast, Hepatocellular, Lung NSC |
| | | |

(continued)

| Peak region | Length (Mb) | Cancer types identified in this analysis but not prior publications |
|---|---|---|
| chr13: 1-23902184 | 13q12.11 | Breast, Lung SC, Ovarian |
| chr13: 46362859-48209064 | 13q14.2 | Hepatocellular, Lung SC, Myeloproliferative disorder, Prostate |
| chr13: 92308911-94031607 | 13q31.3 | Breast, Hepatocellular, Lung NSC, Renal |
| chr14: 1-29140968 | 14q11.2 | Acute lymphoblastic leukemia, Esophageal adenocarcinoma, Myeloproliferative disorder |
| chr14: 65275722-67085224 | 14q23.3 | Dedifferentiated liposarcoma, Myeloproliferative disorder |
| chr14: 80741860-106368585 | 14q32.12 | Acute lymphoblastic leukemia, Dedifferentiated liposarcoma, Melanoma, Myeloproliferative disorder |
| chr15: 1-24740084 | 15q11.2 | Acute lymphoblastic leukemia, Breast, Esophageal adenocarcinoma, Lung NSC, Myeloproliferative disorder, Ovarian |
| chr15: 35140533-43473382 | 15q15.1 | Esophageal adenocarcinoma, Lung NSC, Myeloproliferative disorder |
| chr16: 1-359092 | 16p13.3 | Esophageal adenocarcinoma, Hepatocellular, Lung NSC, Renal |
| chr16: 31854743-53525739 | 116q11.2 | Breast, Hepatocellular, Lung NSC, Melanoma, Renal |
| chr16: 5062786-7709383 | 116p13.3 | Hepatocellular, Lung NSC, Medulloblastoma, Melanoma, Myeloproliferative disorder, Ovarian, Renal |
| chr16: 76685816-78205652 | 16q23.1 | Breast, Colorectal, Esophageal adenocarcinoma, Hepatocellular, Lung NSC, Lung SC, Medulloblastoma, Renal |
| chr16: 80759878-82408573 | 16q23.3 | Colorectal, Hepatocellular, Renal |
| chr16: 88436931-88827254 | 16q24.3 | Colorectal, Hepatocellular, Lung NSC, Prostate, Renal |
| chr17: 10675416-12635879 | 117p12 | Lung NSC, Lung SC, Myeloproliferative disorder |
| chr17: 26185485-27216066 | 117q11.2 | Breast, Colorectal, Dedifferentiated liposarcoma, Lung NSC, Lung SC, |

(continued)

| Peak region | Length (Mb) | Cancer types identified in this analysis but not prior publications |
|---|---|---|
| | | Melanoma, Myeloproliferative disorder, |
| | | Ovarian |
| chr17: 37319013-37988602 | 17q21.2 | Breast, Colorectal, Dedifferentiated |
| | | liposarcoma, Lung SC, Melanoma, |
| | | Myeloproliferative disorder, Ovarian |
| chr17: 7471230-7717938 | 17p13.1 | Lung SC, Myeloproliferative disorder |
| | | |
| chr17: 78087533-78774742 | 17q25.3 | Colorectal, Myeloproliferative disorder |
| | | |
| chr18: 1-587750 | 18p11.32 | Myeloproliferative disorder |
| chr18: 46172638-49935241 | 18q21.2 | Esophageal adenocarcinoma, Lung NSC |
| | | |
| chr18: 75796373-76117153 | 118q23 | Colorectal, Esophageal adenocarcinoma, |
| | | Esophageal squamous, Ovarian, Prostate |
| chr19: 1-526082 | 19p13.3 | Hepatocellular, Lung NSC, Renal |
| chr19: 21788507-34401877 | 119p12 | Hepatocellular, Lung NSC, Renal |
| | | |
| chr19: 52031294-53331283 | 119q13.32 | Breast, Hepatocellular, Lung NSC, |
| | | Medulloblastoma, Ovarian, Renal |
| chr19: 63402921-63811651 | 19q13.43 | Breast, Colorectal, Dedifferentiated |
| | | liposarcoma, Hepatocellular, Lung NSC, |
| | | Medulloblastoma, Ovarian, Renal |
| chr20: 1-325978 | 20p13 | Breast, Dedifferentiated liposarcoma, Lung |
| | | NSC |
| chr20: 14210829-15988895 | 20p12.1 | Esophageal adenocarcinoma, Lung NSC, |
| | | Medulloblastoma, Melanoma, |
| | | Myeloproliferative disorder, Prostate, |
| | | Renal |
| chr21: 38584860-42033506 | 21q22.2 | Breast |
| | | |
| chr22: 20517661-21169423 | 22q11.22 | Acute lymphoblastic leukemia, Esophageal adenocarcinoma |
| chr22: 45488286-49691432 | 22q13.33 | Breast, Hepatocellular, Lung NSC, Lung |
| | | SC |
| chrX: 1-3243111 | Xp22.33 | Esophageal adenocarcinoma, Lung NSC, |
| | | Lung SC |

(continued)

| Peak region | Length (Mb) | Cancer types identified in this analysis but not prior publications |
|---|---|---|
| chrX: 31041721-34564697 | Xp21.2 | Acute lymphoblastic leukemia, Esophageal |
| | | adenocarcinoma, Glioma |

The aneuploidies identified as characteristic of various cancers (e.g., the aneuploidies identified in Tables 3 and 4) may contain genes known to be implicated in cancer etiologies (e.g., tumor suppressors, oncogenes, etc.). These aneuploidies can also be probed to identify relevant but previously unknown genes.

Table 5 illustrates target genes known to be within the identified amplified segment and predicted genes, and Table 6 illustrates target genes known to be within the identified deleted segment and predicted genes.

Table 5 Illustrative, but non-limiting chromosomal segments and genes known or predicted to be present in regions characterized by amplification in various cancers

| Chromosome and band | Peak region | # genes | Known target | Grail top target |
|---|---|---|---|---|
| | | | | |
| 8q24.21 | chr8: 128774432-128849112 | 1 | MFC | MYC |
| 11q13.2 | chr11: 69098089-69278404 | 3 | CCND1 | ORAOV1 |
| 17q12 | chr17: 35067383-35272328 | 6 | ERBB2 | ERBB2, C17orf37 |
| 12q14.1 | chur12: 56419524-56488685 | 7 | CDK4 | TSPAN31 |
| 14q13.3 | chr14: 35708407-36097605 | 3 | NKX2-1 | NKX2-1 |
| 12q15 | chr12: 67440273-67566002 | 1 | MDM2 | MDM2 |
| 7p11.2 | Chr7: 54899301-55275419 | 1 | EGFR | EGFR |
| 1q21.2 | chr1: 148661965-149063439 | 9 | MCL1≠ | MCL1 |
| 8p12 | chr8: 38252951-38460772 | 3 | FGFR1 | FGFR1 |
| 12p12.1 | chr12: 25189655-25352305 | 2 | KRAS | KRAS |
| 19q12 | chr19: 34975531-35098303 | 1 | CCNE1 | CCNE1 |
| 22q11.21 | chr22: 19172385-19746441 | 1 | CRKL | CRKL |
| 12q15 | chr12: 68249634-68327233 | 2 | | LRRC10 |

(continued)

| Chromosome and band | Peak region | # genes | Known target | Grail top target |
|---|---|---|---|---|
| 12q14.3 | chr12: 64461446-64607139 | 1 | HMGA2 | HMGA2 |
| Xq28 | chrX: 152729030-154913754 | 53 | | SPRY3 |
| 5p15.33 | chr5: 1212750-1378766 | 3 | TERT | TERT |
| 3q26.2 | chr3: 170024984-173604597 | 22 | PRKCI | PRKCI |
| 15q26.3 | chr15: 96891354-97698742 | 4 | IGF1R | IGF1R |
| 20q13.2 | chr20: 51603033-51989829 | 1 | | ZNF217 |
| 8p11.21 | chr8: 42006632-42404492 | 6 | | PLAT |
| 1p34.2 | chr1: 39907605-40263248 | 7 | MYCL1 | MYCL1 |
| 17q21.33 | chr17: 44673157-45060263 | 4 | | NGFR, PHB |
| 2p24.3 | chr2: 15977811-16073001 | 1 | MYCN | MYCN |
| 7q21.3 | chr7: 89924533-98997268 | 62 | CDK6 | CDK6 |
| 13q34 | chr13: 108477140-110084607 | 4 | | IRS2 |
| 11q14.1 | chr11: 76699529-78005085 | 14 | | GAB2 |
| 20q13.33 | chr20: 61329497-62435964 | 38 | | BIRC7 |
| 17q23.1 | chr17: 55144989-55540417 | 5 | | RPS6KB1 |
| 11p12 | chr1:119996566-120303234 | 5 | | REG4 |
| 8q21.13 | chr8: 81242335-81979194 | 3 | | ZNF704, ZBTB10 |
| 6p21.1 | chr6: 43556800-44361368 | 18 | | VEGFA |
| 5p11 | chr5: 45312870-49697231 | 0 | | |

(continued)

| Chromosome and band | Peak region | # genes | Known target | Grail top target |
|---|---|---|---|---|
| 20q11.21 | chr20: 29526118-29834552 | 5 | BCL2L1≠ | BCL2L1, ID1 |
| 6q23.3 | chr6: 135561194-135665525 | 1 | MYB** | hsa-mir-548a-2 |
| 1q44 | chr1: 241364021-247249719 | 71 | | AKT3 |
| 5q35.3 | chr5: 174477192-180857866 | 92 | | FLT4 |
| 7q31.2 | chr7: 115981465-11667695₃ | 3 | MET | MET |
| 18q11.2 | chr18: 17749667-22797232 | 21 | | CABLES1 |
| 17q25.1 | chr17: 70767943-171305641 | 13 | | GRB2, ITGB4 |
| 1p32.1 | chr1: 58658784-60221344 | 7 | JUN | JUN |
| 17q11.2 | chr17: 24112056-124310787 | 5 | | DHRS13, FLOT2, ERAL1, PHF12 |
| 17p11.2 | chr17: 18837023-19933105 | 12 | | MAPK7 |
| 8q24.11 | chr8: 116186189-120600761 | 13 | | NOV |
| 12q15 | chr12: 66458200-66543552 | 0 | | |
| 19q13.2 | chr19: 43177306-45393020 | 60 | | LGALS7, DYRK1B |
| 11q22.2 | chr11: 101433436-102134907 | 8 | BIRC2, YAP1 | BIRC2 |
| 4q12 | chr4: 54471680-55980061 | 7 | PDGFRA, KIT | KDR, KIT |
| 12p11.21 | chr12: 30999223-32594050 | 9 | | DDX11, FAM60A |
| 3q28 | chr3: 178149984-199501827 | 143 | PIK3CA | PIK3CA |
| 1p36.33 | Chr1: 1-5160566 | 77 | | TP73 |

(continued)

| Chromosome and band | Peak region | # genes | Known target | Grail top target |
|---|---|---|---|---|
| 17q24.2 | chr17: 62318152-63890591 | 12 | | BPTF |
| 1q23.3 | chr1: 158317017-159953843 | 52 | | PEA15 |
| 1q24.3 | chr1: 169549478-170484405 | 6 | | BAT2D1, MYOC |
| 8q22.3 | chr8: 101163387-103693879 | 14 | | RRM2B |
| 13q31.3 | chr13: 89500014-93206506 | 3 | | GPC5 |
| 12q21.1 | chr12: 70849987-70966467 | 0 | | |
| 12p13.33 | chr12: 1-1311104 | 10 | | WNK1 |
| 12q21.2 | chr12: 76852527-77064746 | 0 | | |
| 1q32.1 | chr1: 201678483-203358272 | 21 | MDM4 | MDM4 |
| 19q13.42 | chr19: 59066340-59471027 | 19 | | PRKCG, TSEN34 |
| 12q12 | chr12: 38788913-42596599 | 12 | | ADAMTS20 |
| 12q23.1 | chr12: 95089777-95350380 | 2 | | ELK3 |
| 12q21.32 | chr12: 85072329-85674601 | 0 | | |
| 10q22.3 | chr10: 74560456-82020637 | 46 | | SFTPA1B |
| 3p11.1 | chr3: 86250885-95164178 | 8 | | POU1F1 |
| 17q11.1 | chr17: 22479313-22877776 | 1 | | WSB1 |
| 8q24.3 | chr8: 140458177-146274826 | 97 | | PTP4A3, MAFA, PARP10 |
| Xq12 | chrX: 66436234-67090514 | 1 | AR | AR |

(continued)

| Chromosome and band | Peak region | # genes | Known target | Grail top target |
|---|---|---|---|---|
| 16q12 | chr6: 63255006-65243766 | 3 | | PTP4A1 |
| 114q11.2 | chr14: 1-23145193 | 95 | | BCL2L2 |
| 9q34.3 | chr9: 137859478-140273252 | 76 | | NRARP, MRPL41, TRAF2, LHX3 |
| 6p24.1 | chr6: 1-23628840 | 95 | | E2F3 |
| 13q12.2 | chr13: 1-40829685 | 110 | | FOXO1 |
| 12q21.1 | chr12: 72596017-73080626 | 0 | | |
| 14q32.33 | chr14: 106074644-106368585 | 0 | | |
| 11p13 | chr11: 32027116-37799354 | 35 | | WT1 |

**Table 6 Illustrative, but non-limiting chromosomal segments and genes known or predicted to be present in regions characterized by amplification in various cancers**

| Chromosome and band | Peak region | # genes | Known target | Grail top target |
|---|---|---|---|---|
| | | | | |
| 9p21.3 | chr9: 21489625-22474701 | 5 | CDKN2A/B | DKN2A |
| 3p14.2 | chr3: 58626894-61524607 | 2 | FHIT§ | FHIT |
| 16q23.1 | chr16: 76685816-78205652 | 2 | WWOX§ | WWOX |
| 9p24.1 | chr9: 7161607-12713130 | 3 | PTPRD§ | PTPRD |
| 20p12.1 | chr20: 14210829-15988895 | 2 | MACROD2§ | FLRT3 |
| 6q26 | chr6: 161612277-163134099 | 1 | PARK2§ | PARK2 |
| 13q14.2 | chr13: 46362859-48209064 | 8 | RB1 | RB1 |
| 2q22.1 | chr2: 138479322-143365272 | 3 | LRP1B§ | LRP1B |

(continued)

| Chromosome and band | Peak region | # genes | Known target | Grail top target |
|---|---|---|---|---|
| 4q35.2 | chr4: 186684565-191273063 | 15 | | FRG2, |
| | | | | TUBB4Q |
| 15q11.2 | chr5: 57754754-59053198 | 5 | PDE4D§ | PLK2, |
| | | | | PDE4D |
| 16p13.3 | chr16: 5062786-7709383 | 2 | A2BP1§ | A2BP1 |
| | | | | |
| 7q34 | chr7: 141592807-142264966 | 3 | TRB@{circumflex over ()} | PRSS1 |
| | | | | |
| 2q37.3 | chr2: 241477619-242951149 | 119 | | TMEM16G, |
| | | | | ING5 |
| 19p13.3 | chr19: 1-526082 | 10 | | GZMM, |
| | | | | THEG, |
| | | | | PPAP2C, |
| | | | | C19orf20 |
| 10q23.31 | chr10: 89467202-90419015 | 4 | PTEN | PTEN |
| | | | | |
| 8p23.2 | chr8: 2053441-6259545 | 1 | CSMD1§ | CSMD1 |
| | | | | |
| 1p36.31 | chr1: 3756302-6867390 | 23 | | DFFB, |
| | | | | ZBTB48, |
| | | | | AJAP1 |
| 4q22.1 | chr4: 91089383-93486891 | 2 | | MGC48628 |
| | | | | |
| 18q23 | chr18: 75796373-76117153 | 4 | | PARD6G |
| | | | | |
| 6p25.3 | chr6: 1543157-2570302 | 2 | | FOXC1 |
| | | | | |
| 19q13.43 | chr19: 63402921-63811651 | 17 | | ZNF324 |
| | | | | |
| Xp21.2 | chrX: 31041721-34564697 | 2 | DMD§ | DMD |
| | | | | |
| 11q25 | chr11: 130280899-134452384 | 12 | OPCML§, | HNT |
| | | | HNT§ | |
| 13q12.11 | chr13: 1-23902184 | 29 | | LATS2 |
| 22q13.33 | chr22: 45488286-49691432 | 38 | | TUBGCP6 |
| | | | | |

(continued)

| Chromosome and band | Peak region | # genes | Known target | Grail top target |
|---|---|---|---|---|
| 15q11.2 | chr15: 1-24740084 | 20 | | A26B1 |
| 22q11.22 | chr22: 20517661-21169423 | 3 | | VPREB1 |
| 10q26.3 | chr10: 129812260-135374737 | 35 | | MGMT, SYCE1 |
| 12p13.2 | chr12: 11410696-12118386 | 2 | ETV6$ | ETV6 |
| 8p23.3 | chr8: 1-392555 | 2 | | ZNF596 |
| 1p36.11 | chr1: 26377344-27532551 | 24 | | SFN |
| 11p15.5 | chr11: 1-1391954 | 49 | | RASSF7 |
| 17q11.2 | chr17: 26185485-27216066 | 10 | NF1 | NF1 |
| 11q23.1 | chr11: 107086196-116175885 | 61 | ATM | CADM1 |
| 9p24.3 | chr9: 1-708871 | 5 | | FOXD4 |
| 10q11.23 | chr10: 52313829-53768264 | 4 | PRKG1§ | DKK1, PRKG1 |
| 15q15.1 | chr15: 35140533-43473382 | 109 | | TUBGCP4 |
| 1p13.2 | chr1: 110339388-119426489 | 81 II | | MAGI3 |
| Xp22.33 | chrX: 1-3243111 | 21 | | SHOX |
| 3p26.3 | chr3: 1-2121282 | 2 | | CHL1 |
| 9p13.2 | chr9: 36365710-37139941 | 2 | PAX5 | MELK |
| 17p13.1 | chr17: 7471230-7717938 | 10 | TP53 | ATP1B2 |
| 12q24.33 | chr12: 131913408-132349534 | 7 | | CHFR |
| 7q36.3 | chr7: 156893473-158821424 | 7 | PTPRN2§ | NCAPG2 |
| 6q16.1 | chr6: 76630464-105342994 | 76 | | FUT9, C6orf165, C6orf162, GJA10 |

(continued)

| Chromosome and band | Peak region | # genes | Known target | Grail top target |
|---|---|---|---|---|
| 5q21.1 | chr5: 85837489-133480433 | 142 | APC | APC |
| 8p11.22 | chr8: 39008109-41238710 | 7 | | C8orf4, ZMAT4 |
| 19q13.32 | chr19: 52031294-53331283 | 25 | | BBC3 |
| 10p15.3 | chr10: 1-1042949 | 4 | | TUBB8 |
| 1p31.1 | chr1: 71284749-74440273 | 4 | NEGR1§ | NEGR1 |
| 13q31.3 | chr13: 92308911-94031607 | 2 | GPC6§ | GPC6, DCT |
| 16q11.2 | chr16: 31854743-53525739 | 37 | | RBL2 |
| 20p13 | chr20: 1-325978 | 10 | | SOX12 |
| 5q35.3 | chr5: 177541057-180857866 | 43 | | SCGB3A1 |
| 1q43 | chr1: 223876038-247249719 | 173 | RYR2§ | FH, ZNF678 |
| | chr16: 1-359092 | 16 | | HBZ |
| 17q21.2 | chr17: 37319013-37988602 | 22 | | CNP |
| 2p25.3 | chr2: 1-15244284 | 51 | | MYT1L |
| 3q13.31 | chr3: 116900556-120107320 | 1 | | LSAMP |
| 7q21.11 | chr7: 65877239-79629882 | 73 | MAGI2§ | CLDN4 |
| 7q35 | chr7: 144118814-148066271 | 3 | CNTNAP2§ | CNTNAP2 |
| 14q32.12 | chr14: 80741860-106368585 | 154 | | PRIMA1 |
| 16q24.3 | chr16: 88436931-88827254 | 9 | | C16orf3 |
| 3q26.31 | chr3: 175446835-178263192 | 1 | NAALADL2§ | NAALADL2 |
| 17q25.3 | chr17: 78087533-78774742 | 8 | | ZNF750 |

(continued)

| Chromosome and band | Peak region | # genes | Known target | Grail top target |
|---|---|---|---|---|
| 19p12 | chr19: 21788507-34401877 | 12 | | ZNF492, |
| | | | | ZNF99 |
| 12q23.1 | chr12: 97551177-99047626 | 3 | ANKS1B§ | ANKS1B |
| | | | | |
| 4p16.3 | chr4: 1-435793 | | | ZNF141 |
| 18p11.32 | chr18: 1-587750 | 4 | | COLEC12 |
| 2q33.2 | chr2: 204533830-206266883 | 1 | PARD3B§ | PARD3B |
| | | | | |
| 8p21.2 | chr8: 22125332-30139123 | 63 | | DPYSL2, |
| | | | | STMN4 |
| 8q11.22 | chr8: 42971602-72924037 | 86 | SNTG1§ | FLJ23356, |
| | | | | ST18, |
| | | | | RB1CC1 |
| 16q23.3 | chr16: 80759878-82408573 | 2 | CDH13§ | CDH13 |
| | | | | |
| 11q14.1 | chr11: 82612034-85091467 | 6 | DLG2§ | CCDC89, |
| | | | | CCDC90B, |
| | | | | TMEM126A |
| 14q23.3 | chr14: 65275722-67085224 | 7 | | GPHN, |
| | | | | MPP5 |
| 7p22.2 | chr7: 3046420-4279470 | 1 | SDK1§ | SDK1 |
| | | | | |
| 13q34 | chr13: 111767404-114142980 | 25 | | TUBGCP3 |
| | | | | |
| 17p12 | chr17: 10675416-12635879 | 5 | MAP2K4 | MAP2K4, |
| | | | | ZNF18 |
| 21q22.2 | chr21: 38584860-42033506 | 19 | DSCAM§, | DSCAM |
| | | | TMPRSS2/ERG$ | |
| 18q21.2 | chr18: 46172638-49935241 | 7 | SMAD4, | DCC |
| | | | DCC§ | |
| 6q22.1 | chr6: 101000242-121511318 | 87 | | GTF3C6, |
| | | | | TUBE1, |
| | | | | ROS1 |
| 14q11.2 | chr14: 1-29140968 | 140 | | ZNF219, |
| | | | | NDRG2 |

Although the examples herein concern humans and the language is primarily directed to human concerns, the concepts described herein are applicable to genomes from any plant or animal.

In various embodiments, it is contemplated to use the method identified herein to identify CNVs of segments comprising the amplified regions or genes identified in Table 5 and/or to use the methods identified herein to identify CNVs of segments comprising the deleted regions or genes identified in Table 6. In other embodiments, it is contemplated to use the method identified herein to screen for the presence of CNVs of segments that were not previously linked to cancer or are not described in Table 5 or 6.

In one embodiment, the methods described herein provide a means to assess the association between gene amplification and the extent of tumor evolution. Correlation between amplification and/or deletion and stage or grade of a cancer may be prognostically important because such information may contribute to the definition of a genetically based tumor grade that would better predict the future course of disease with more advanced tumors having the worst prognosis. In addition, information about early amplification and/or deletion events may be useful in associating those events as predictors of subsequent disease progression.

Gene amplification and deletions as identified by the method can be associated with other known parameters such as tumor grade, histology, Brd/Urd labeling index, hormonal status, nodal involvement, tumor size, survival duration and other tumor properties available from epidemiological and biostatistical studies. For example, tumor DNA to be tested by the method could include atypical hyperplasia, ductal carcinoma in situ, stage I-III cancer and metastatic lymph nodes in order to permit the identification of associations between amplifications and deletions and stage. The associations made may make possible effective therapeutic intervention. For example, consistently amplified regions may contain an overexpressed gene, the product of which may be able to be attacked therapeutically (for example, the growth factor receptor tyrosine kinase, p185HER2).

In various embodiments, the method described herein can be used to identify amplification and/or deletion events that are associated with drug resistance by determining the copy number variation of nucleic acid sequences from primary cancers to those of cells that have metastasized to other sites. If gene amplification and/or deletion is a manifestation of karyotypic instability that allows rapid development of drug resistance, more amplification and/or deletion in primary tumors from chemoresistant patients than in tumors in chemosensitive patients would be expected. For example, if amplification of specific genes is responsible for the development of drug resistance, regions surrounding those genes would be expected to be amplified consistently in tumor cells from pleural effusions of chemoresistant patients but not in the primary tumors. Discovery of associations between gene amplification and/or deletion and the development of drug resistance may allow the identification of patients that will or will not benefit from adjuvant therapy.

In other embodiments, the method described herein can be used to identify the presence of genome-wide instability and/or microsatellite instability and/or specific combinations of Copy Number Variations (which could span whole chromosomes, chromosome arms, or smaller DNA segments).

In yet another embodiment, the method described herein can be used to identify the tumor origin, i.e. the primary tissue or organ where the tumor originated from prior to becoming metastatic.

Both complete and partial chromosomal aneuploidies as well as smaller Copy Number Variations of DNA segments that could be associated with the formation, and progression of cancer can be determined according to the present method.

## II Sequencing, aligning and correction

As mentioned above, only a fraction of the genome is sequenced. In one aspect, even when a pool of nucleic acids in a specimen is sequenced at <100% genomic coverage instead of at several folds of coverage, and among the proportion of sequenced nucleic acid molecules, most of each nucleic acid species is not sequenced or sequenced only once.

This is contrasted from situations where targeted enrichment is performed of a subset of the genome prior to the sequencing reaction, followed by high-coverage sequencing of that subset.

In one embodiment, massive parallel short-read sequencing is used.. Short sequence tags or reads are generated, e.g. from a certain length between 20bp and 400bp. Alternatively, paired end sequencing could be performed.

In an embodiment, a pre-processing step is available for pre-processing the obtained reads. Such pre-processing option allows filtering low quality reads, thereby preventing them from being mapped. Mapping of low quality reads can require prolonged computer processing capacity, can be incorrect and risks increasing the technical noise in the data, thereby obtaining a less accurate parameter. Such pre-processing is especially valuable when using next-generation sequencing data, which has an overall lower quality or any other circumstance which is linked to an overall lower quality of the reads.

The generated reads can subsequently be aligned to one or more human reference genome sequences. Preferably, the number of aligned reads are counted and/or sorted according to their chromosomal location.

An additional clean-up protocol can be performed, whereby deduplication is performed, e.g. with Picard tools, retaining only uniquely mapped reads. Reads with mismatches and gaps can be removed. Reads that map to blacklisted regions can be excluded. Such blacklisted regions may be taken from a pre-defined list of e.g. common CNVs, collapsed repeats, DAC blacklisted regions as identified in the ENCODE project (i.e. a set of regions in the human genome that have anomalous, unstructured, high signal/read counts in NGS experiments independent of cell line and type of experiment) and the undefined segment of the reference genome. In one embodiment, blacklisted regions are provided to the user. In another embodiment, the user may use or define his or her own set of blacklisted regions.

In a further embodiment, chromosomes are divided into regions of a predefined length, generally referred to as bins. In an embodiment, the bin size is a pre-defined size provided to the user. In another embodiment, said bin size can be defined by a user, can be uniformly for all chromosomes or can be a specific bin size per chromosome or can vary according to the obtained sequence data. Change of the bin size can have an effect on the final parameter to be defined, either by improving the sensitivity (typically obtained by decreasing the bin size, often at the cost of the specificity) or by improving the specificity (generally by increasing the bin size, often at the cost of the sensitivity). A possible bin size which provides an acceptable specificity and sensitivity is 50 kb.

In a further step, the aligned and filtered reads within a bin are counted, in order to obtain read counts.

The obtained read counts can be corrected for the GC count for the bin. GC bias is known to aggravate genome assembly. Various GC corrections are known in the art (e.g. Benjamini et al., Nucleic Acid Research 2012). In a preferred embodiment, said GC correction will be a LOESS regression. In one embodiment, a user of the methodology according to the current invention can be provided with the choice of various possible GC corrections.

In a subsequent step, the genomic representation (GR) of read counts per bin is calculated. Such representation is preferably defined a ratio between the GC-corrected read counts for a specific bin and the sum of all GC-corrected read counts.

In an embodiment, said GR is defined as follows: $GRi = \frac{GCi}{\sum_k GCk} \cdot 10^7$ with k over all chromosomal bins

(whereby the factor $10^7$ in the above formula is arbitrary defined, and can be any constant value)

In a final step, the obtained GR per bin are aggregated over a region, whereby said region may be a subregion (window) of a chromosome or the full chromosome. Said window may have a predefined or variable size, which can optionally be chosen by the user. A possible window could have a size of 5 MB or 100 adjacent bins of size 50 kb.

The GR aggregated for a chromosome can be defined by

$$GRi = \frac{\sum_{j \in (bins\ on\ chr)} GCi}{\sum_{k \in (all\ bins)GCk} GCk}$$

In a further embodiment, the genomic representation of a set of reference samples shall be calculated. Said set of reference samples (or also termed reference set) can be predefined or chosen by a user (e.g. selected from his/her own reference samples). By allowing the user the use of an own reference set, a user will be enabled to better capture the recurrent technical variation of his/her environment and its variables (e.g. different wet lab reagents or protocol, different NGS instrument or platform, etc.) In a preferred embodiment, said reference set comprises genomic information of 'healthy' samples, that are known to not contain (relevant) aneuploidies. The genomic representation (GR) of the reference set can be defined, either at the genome level and/or at a subregion (chromosome, chromosomal segment, window, or bin).

Other single molecule sequencing strategies such as that by the Roche 454 platform, the Applied Biosystems SOLiD platform, the the Helicos True Single Molecule DNA sequencing technology, the single molecule, real-time (SMRT™) technology of Pacific Biosciences, and nanopore sequencing technologies like MinION, GridION or PromethION from Oxford Nanopore Technologies could similarly be used in this application.

III Determining scores, parameter and secondary parameters

From the alignments and the obtained read counts or a derivative thereof, optionally corrected for GC content and/or total number of reads obtained from said sample, scores are calculated which eventually lead to a parameter allowing the determination of the presence of an aneuploidy in a sample. Said scores are normalized values derived from the read counts or mathematically modified read counts, whereby normalization occurs in view of the reference set. As such, each score is obtained by means of a comparison with the reference set. The term first score is used to refer to score linked to the read count for a target chromosome or a chromosomal segment. A collection of scores is a set of scores derived from a set of normalized number of reads that may include the normalized number of reads of said target chromosomal segment or chromosome.

Preferably, said first score represents a Z score or standard score for a target chromosome or chromosomal segment. Preferably, said collection is derived from a set of Z scores obtained from a corresponding set of chromosomes or chromosomal segments that include said target chromosomal segment or chromosome.

Such scores can be calculated as follows:

$$Zi = \frac{GRi - \mu\,ref,i}{\sigma\,ref,i}$$

With i a window or a chromosome or a chromosome segment.

A summary statistic of said collection of scores can e.g. be calculated as the mean or median value of the individual scores.

Another summary statistic of said collection of scores can be calculated as the standard deviation or median absolute deviation or mean absolute deviation of the individual scores.

Optionally but not necessarily, the same collection of scores is used for both types of calculations.

Said parameter p will be calculated as a function of the first score and a derivative (e.g. summary statistic) of the collection of scores. In a preferred embodiment, said parameter p will be a ratio between the first score corrected by the collection of scores (or a derivative thereof) and a derivative of said collection of scores.

In another embodiment, said parameter will be a ratio between the first score corrected by a summary statistic of a first collection of scores and a summary statistic of a different, second collection of scores, in which both collections of scores include the first score.

In a specifically preferred embodiment, said parameter p is a ratio between the first score, corrected by a summary statistic of said collection of scores, and a summary statistic of said collection of scores. Preferably, the summary statistic is selected from the mean, median, standard deviation, median absolute deviation or mean absolute deviation. In one embodiment, said both used summary statistics in the function are the same. In another, more preferred embodiment, said summary statistics of the collection of scores differ in the numerator and denominator.

Typically, a suitable embodiment according to the current invention involves the following steps (after having obtained DNA sequences from a random sequencing process on a biological sample).

- aligning said obtained sequences to a reference genome;

- counting the number of reads on a set of chromosomal segments and/or chromosomes thereby obtaining read counts;

- normalizing said read counts or a derivative thereof into a normalized number of reads;

- obtaining a first score and a collection of scores of said normalized reads, whereby said first score is derived from the normalized reads for a target chromosome or chromosomal segment and said collection of scores is a set of scores derived from a corresponding set of chromosomes or chromosome segments that include said target chromosomal segment or chromosome;

- calculating a parameter p from said first score and said collection of scores, whereby said parameter represents a ratio between

    * said first score, corrected by a summary statistic of said collection of scores, and

    * a summary statistic of said collection of scores.

A possible parameter p can be calculated as follows:

$$Z \text{ of } Z_i = \frac{Z_i - \underset{j=i,a,b,\ldots}{\mathrm{median}}(Z_j)}{\underset{j=i,a,b,\ldots}{\mathrm{sd}}(Z_j)}$$

Whereby Zi represents the first score and Z j the collection of scores and whereby i represents the target chromosome or chromosomal section, and whereby j represents a collection chromosomes or chromosomal segments i, a, b, .. that includes said target chromosomal segment or chromosome i. In another embodiment, said parameter p is calculated as

$$Z \text{ of } Z_i = \frac{Z_i - \underset{j=i,a,b,\ldots}{\mathrm{mean}}\left(Z_j\right)}{\underset{j=i,a,b,\ldots}{\mathrm{mad}}\left(Z_j\right)}$$

Whereby Zi represents the first score and Z j the collection of scores and whereby i represents the target chromosome or chromosomal section, and whereby j represents a collection of chromosomes or chromosomal segments i, a, b, .. that includes said target chromosomal segment or chromosome i.

In yet another, most preferred embodiment, said parameter p is calculated as

$$Z \text{ of } Z_i = \frac{Z_i - \underset{j=i,a,b,\ldots}{\mathrm{median}}\left(Z_j\right)}{\underset{j=i,a,b,\ldots}{\mathrm{mad}}\left(Z_j\right)}$$

Whereby $Z_i$ represents the first score and $Z_j$ the collection of scores and whereby i represents the target chromosome or chromosomal section, and whereby j represents a collection of chromosomes or chromosomal segments i, a, b, .. that includes said target chromosomal segment or chromosome i.

Apart from the parameter p which will allow the identification of the presence of aneuploidies, secondary parameters can be calculated which may serve as quality control or provide additional information with regard to one or more aneuploidies present in the sample.

A first secondary parameter which can be calculated allows defining whether chromosomal and large subchromosomal aneuploidies are present in the sample (compared to e.g. smaller aneuploidies). In a preferred embodiment, such parameter is defined by the median of the Z scores measured per subregions (e.g. 5 Mb windows) in a target chromosome. If more than 50% of these subregions are affected, this will show in the secondary parameters.

In another embodiment, a secondary parameter may be calculated as the median of the absolute value of the Z scores calculated over the remaining chromosomes (that is all chromosomes except the target chromosome or chromosomal segment) per subregion (e.g. 5 Mb windows).The latter secondary parameter allows the detection of the presence of technical or biological instabilities (cf. malignancies, cancer). If less than half of the windows of the other or all autosomes or chromosomes are affected, this secondary parameter will not be affected. If more than 50% of the windows is affected, this will be derivable from said secondary parameters.

In another embodiment, the current invention also provides for a quality score (QS). QS allows to assess the overall variation across the genome. A low QS is an indication of a good sample processing and a low level of technical and biological noise. An increase in the QS can indicate two possible reasons. Either an error occurred during the sample processing. In general, the user will be requested to retrieve and sequence a new biological sample. This is typical for moderately increased QS scores. A strongly increased QS is an indication of a highly aneuploid or genome-wide instable sample and the user may be encouraged to do a confirmatory test to further assess whether the subject is developing cancer. Preferably, said QS is determined by calculating the standard deviations of all Z distributions for the chromosomes and by removing the highest and lowest scoring chromosome.

For instance, samples with a QS exceeding 2 are considered to be of poor quality, or at increased risk for cancer and a QS between 1.5 and 2 are of intermediate quality.

IV. Comparison to cutoff value

The parameter p as calculated in the embodiments above shall subsequently be compared with a cutoff parameter for determining whether a change compared to a reference quantity exists (i.e. an imbalance), for example, with regards to the ratio of amounts of two chromosomal regions (or sets of regions). The presence of an aneuploidy and/or an increased number of said aneuploidy is an indicator of the presence and/or increased risk of a cancer. In one embodiment, the user will be able to define its own cutoff value, either empirically on the basis of experience or previous experiments, or for instance based on standard statistical considerations. If a user would want to increase the sensitivity of the test, the user can lower the thresholds (i.e. bring them closer to 0). If a user would want to increase the specificity of the test, the user can increase the thresholds (i.e. bring them further apart from 0). A user will often need to find a balance between sensitivity and specificity, and this balance is often lab- and application -specific, hence it is convenient if a user can change the threshold values him- or herself.

Based on the comparison with the cutoff value, an aneuploidy may be found present or absent. Such presence is indicative for the presence and/or increased risk of a cancer.

In an embodiment of the current invention, comparison of parameter p with a cutoff value is sufficient for determining the presence or absence of an aneuploidy. In another embodiment, said aneuploidy is determined on the basis of a comparison of parameter p with a cutoff value and a comparison of at least one of the secondary parameters, quality score and/or first score with a cutoff value, whereby for each score a corresponding cutoff value is defined or set.

In a preferred embodiment, said presence/absence of an aneuploidy is defined by a comparison of parameter p with a predefined cutoff value, as well as by comparison of all secondary parameters, quality and first scores as described above with their corresponding cutoff values.

The final decision tree may thus be dependent on parameter p alone, or combined with one of the secondary parameters and/or quality score or first score as described above.

In a preferred embodiment, said methodology according to the current invention comprises the following steps:

- multiplex sequencing of 50 bp single-end reads (performed by end user)

- uploading sequence reads

- mapping of reads to a reference genome

- count number of reads per bin (a bin has a size of 50 kb)

- compute GC content per bin and correct for GC content

- compute Genomic Representation (GR) score per bin. For bin i this equals

$$GRi = \frac{\sum_{j \in (bins\ on\ chr)} GCi}{\sum_{k \in (all\ bins)GCk} GCk}$$

- aggregate the GR values per window (a window consists out of 100 consecutive windows)

- compute a Z score per window or per chromosome, whereby the Z score is based on the GR score per chromosome, compared with the GR scores obtained in a set of reference samples.

$$Z_i = \frac{GR_i - \mu_{\mathrm{Ref},i}}{\sigma_{\mathrm{Ref},i}}$$

with i a chromosome or a window, $\mu_{\mathrm{Ref},i}$ the average or median GR score for the corresponding bins in the set of reference samples and $\sigma_{\mathrm{Ref},I}$ the standard deviation of the GR scores for the corresponding bins in the set of reference samples.

- computing of a ZofZ score, whereby the ZofZ score is based on the Z score, corrected by the median (or mean) of the Z scores of a collection of chromosomes including target chromosome i and divided by a factor that measures the variability of the Z scores of a collection of chromosomes that includes the target chromosome i (standard deviation or a more robust version thereof, like e.g. the median absolute deviation or mad).

- comparison of the Z score with a threshold value, and the ZofZ score with a threshold value, to predict the presence or absence of an aneuploidy.

In a further preferred embodiment, said prediction of the presence or absence of an aneuploidy occurs via a decision tree based on parameter p and secondary parameters.

V Toolbox and kit

By preference, the methodologies as described above are all computer implemented. To that purpose, the current invention equally relates to a computer program product comprising a computer readable medium encoded with a plurality of instructions for controlling a computing system to perform an operation for performing analysis of a chromosomal or subchromosomal aneuploidy in a biological sample obtained from a subject, wherein the biological sample includes nucleic acid molecules.

With regard to the determination of the presence or absence of aneuploidy or genome-wide instability in a sample, the operation comprises the steps of:

- receiving the sequences of at least a portion the nucleic acid molecules contained in a biological sample obtained from said pregnant female;

- aligning said obtained sequences to a reference genome;

- counting the number of reads on a set of chromosomal segments and/or chromosomes thereby obtaining read counts;

- normalizing said read counts or a derivative thereof into a normalized number of reads;

- obtaining a first score of said normalized reads and a collection of scores of said normalized reads, whereby said first score is derived from the normalized reads for a target chromosome or chromosomal segment and whereby said collection of scores is a set of scores derived from the normalized number of reads for a set of chromosomes or chromosomal segments that include said target chromosomal segment or chromosome;

- calculating a parameter p from said first score and said collection of scores.

In a preferred embodiment, said parameter whereby said parameter represents a ratio between

* a first score, corrected by a summary statistics of said collection of scores, and

* a summary statistics of the collection of scores.

Said operations can be performed by a user or practitioner in an environment remote from the location of sample collection and/or the wet lab procedure, being the extraction of the nucleic acids from the biologic sample and the sequencing.

Said operations can be provided to the user by means of adapted software to be installed on a computer, or can be stored into the cloud.

After having performed the required or desired operation, the practitioner or user will be provided with a report or score, whereby said report or score provides information on the feature that has been analyzed. Preferably, report will comprise a link to a patient or sample ID that has been analyzed. Said report or score may provide information on the presence or absence of an aneuploidy in a sample, whereby said information is obtained on the basis of a parameter which has been calculated by the above mentioned methodology. The report may equally provide information on the nature of the aneuploidy (if detected, e.g. large or small chromosomal aberrations) and/or on the quality of the sample that has been analyzed.

It shall be understood by a person skilled in the art that above-mentioned information may be presented to a practitioner in one report.

By preference, above mentioned operations are part of a digital platform which enables molecular analyzing of a sample by means of various computer implemented operations.

In particular, the current invention also comprises a visualization tool, which enables the user or practitioner to visualize the obtained results as well as the raw data that has been imputed in the system. In an embodiment, said visualizations comprises a window per chromosome, depicting the chromosome that has been analyzed, showing the reads per region and the scores and/or parameter that has been calculated. By showing to the practitioner or user the calculated scores and parameter together with the visual depiction of the read counts, a user may perform an additional control or assessment of the obtained results. By allowing the user to look at the data, users will be able to define improved decision rules and thresholds.

Moreover, an additional control is added, as the visual data per chromosome enables the user to evaluate for every chromosome if the automated classification is correct. This adds an additional safety parameter.

In a preferred embodiment, said platform and visualization tool is provided with algorithms which take into account the fact that certain regions give more reads (due to a recurrent technical bias that makes some regions of the genome always over- or under-represented). Correction measures may be provided for this overrepresentation by making a comparison with a reference set (that is ideally processed using the same or similar protocol) and plotting e.g. Z scores or alternative scores that represent the unlikeliness of certain observations under the assumption of euploidy. Standard visualization tools only display read count, and do not allow correcting for the recurrent technical bias.

Finally, based on the link between the obtained scores and/or parameter and the visual data per chromosome a user or practitioner may decide to alter the threshold/cutoff value that is used to define the presence of an aneuploidy.

As such, the user may decide to aim for a higher sensitivity (e.g. being less stringent on the decrease/increase of the parameter or scores) or higher specificity (e.g. by being more stringent on the increase/decrease of parameter or scores).

The platform may be provided with other features, which provide for an accurate analysis of the molecular data obtained from the biological sample.

The platform according to the current invention is inherently compatible with many different types of NGS library preparation kits and protocols and NGS sequencing platform. This is an advantage as a user will not have to invest in dedicated NGS sequencing platform or NGS library preparation kits that are specific for a specific application, but instead a user can use its preferred platform and kit. Moreover, this allows a user a certain degree of flexibility in material to be used. If newer or cheaper instruments or kits become available, a user will be allowed an easy change.

As mentioned before, the current methodology is compatible with cell-free DNA extracted from various sorts of biological samples, including blood, saliva and urine. Using urine or saliva instead of blood would represent a truly non-invasive sample type and allows for e.g. home-testing and shipment of the sample to the test lab. This is obviously an additional advantage compared to other sample obtaining methods such as drawing blood.

The invention is further described by the following non-limiting examples which further illustrate the invention, and are not intended to, nor should they be interpreted to, limit the scope of the invention.

**Examples**

Preparation and sequencing of the sample

1. Blood collection, plasma separation and cell-free DNA extraction

One tube (10 ml) of peripheral blood is collected in Streck tubes and stored at 4°C. The blood is collected via a standard phlebotomy procedure.

The plasma (+/- 5 ml) is separated maximum 72 hours after blood sampling by the standard dual centrifugation method:

- The blood sample is centrifuged at 2000xg for 20 minutes (this may be done at room temperature), without using the brake.

- The plasma is then transferred to either three 1.5ml low binding tubes, or to a single 5 ml low binding tube. A second centrifugation at 13,000xg is done for 2 minutes (this may be done at room temperature).

- The plasma is transferred to sterile 1.5 ml or 5 ml low binding tubes for storage at -20°C prior to cell-free DNA (cfDNA) extraction.

Optionally, the buffy coat layer can be stored for future testing. Genomic DNA from the buffy coat layer can be investigated to confirm or exclude germline abnormalities.

The cell-free DNA is extracted from the plasma using the QIAamp Circulating Nucleic Acid Kit (Qiagen) according to the manufacturer's recommendations, with a final elution volume of 60 $\mu$l. The DNA samples are stored at -20°C when not used immediately for library preparation.

2. cfDNA quantification

The extracted cfDNA is quantified using a Qubit fluorometer. The cell-free DNA concentration is usually around 0.1-1 ng/$\mu$l.

3. Library preparation

25 $\mu$l of the extracted cfDNA is used as starting material for library preparation.

During library preparation, the DNA samples are adapted for next-generation sequencing. Adaptors are added to the ends of the DNA fragments.

The sequencing libraries are prepared using the TruSeq ChIP library preparation kit (Illumina) with some adaptation of the manufacturer's protocol by reducing the reagent volumes to allow the generation of sequencing libraries using low starting amounts of DNA.

The library preparation protocol may be summarized as follows:

(Note: The bead-based size selection for removal of large DNA fragments and

removal of small DNA fragments described in the protocol is NOT used.)

_End repair of the DNA fragments:_

1. Add 5 μl Resuspension Buffer and 20 μl End Repair Mix to the 25 μl starting material (total = 50 μl)
The bead-based size selection for removal of large DNA fragments and removal of small DNA fragments described in the protocol is NOT used.

2. Incubate 30 minutes at 30°C

3. Add 80 μl undiluted AMPure beads to the 50 μl sample mixture after end repair.

4. Wash the beads twice with 190 μl 80% EtOH

5. Resuspend the dried pellet with 10 μl Resuspension Buffer. Transfer 9 μl of the supernatant to a new tube.

_Adenylation of the the 3' ends_

1. Add 6.25 μl A-tailing Mix

2. Heat 30 minutes at 37°C + 5 minutes at 70°C

_Ligation of the indexed paired-end adaptors to the DNA_

1. Adaptors are diluted 1/2 with Resuspension Buffer => add 2,5 μl to sample

2. Add 1.25 μl Ligation Mix (no Resuspension Buffer). Incubate 30 minutes at 30°C

3. Add 2.5 μl Stop ligation buffer

4. Add 21 μl AMPure beads for clean-up

5. Wash the beads twice with 190 μl 80% EtOH

6. Resuspend the dried pellet in 27 μl Resuspension Buffer. Transfer 25 μl of the supernatant to a new tube.

7. Add 25 μl AMPure beads for clean-up

8. Wash the beads twice with 190 μl 80% EtOH

9. Resuspend the dried pellet in 12.5 μl Resuspension Buffer. Transfer 10 μl of the supernatant to a new tube.

_Enrich DNA fragments_

1. Prepare the PCR mix by mixing 2,5 μl PCR Primer Cocktail and 12,5 μl PCR Master Mix for each sample.

2. PCR conditions:

98°C for 30 seconds

15 cycles of:

    98°C for 10 seconds

    60°C for 30 seconds

    72°C for 30 seconds

    72°C for 5 minutes

    hold at 4°C

3. Add 25 μl of AMPure beads for clean up

4. Wash the beads twice with 190 μl 80% EtOH

5. Resuspend the dried pellet in 32.5 μl Resuspension Buffer. Transfer 30 μl of the supernatant to a new tube.

6. Use 2 μl of the sample for Qubit quantification and 2μl for fragment analysis (see next section)

<u>4. Library quality check</u>

Proper cell-free DNA isolation and NGS library preparation are tested by analyzing every library on the Fragment Analyzer (Advanced Analytical Technologies Inc., Germany) prior to sequencing, to assess:

• the size distribution (confirm suitable size profile using concentration, peak ratio, peak height, ...),

• the quality of the library. Samples containing high molecular weight fragments will be classified as not eligible for sequencing (indicates contamination with genomic DNA).

Typical libraries demonstrate a narrow size distribution with a peak at about 300-350 bp.

Additionally a Qubit quantification step is performed so that the enrichment reaction is done with the appropriate amount of input DNA material. DNA concentration is usually around 15-30 ng/μl.

<u>5. Normalize and pool libraries</u>

Samples are indexed during library preparation and up to 24 samples are normalized and pooled in equal volumes for multiplex sequencing across both lanes of an Illumina HiSeq2500 flow cell.

<u>6. NGS run</u>

Sequencing is performed on the HiSeq 2500 (Illumina) in Rapid Run mode producing 50bp single end reads.

<u>Detection of aneuploidy in a biological sample: validation of the methodology</u>

* Mapping and filtering of the mapped reads

The 50bp single end sequence reads of a test sample are mapped to the reference genome GRCh37.75 with BWA-backtrack. With Picard tools duplicated reads can be removed and based on the mapping quality reads mapping to multiple locations can be discarded. Also reads mapping sub-optimally to multiple locations are removed. To reduce sample variability, we retain only those reads that match perfectly with the reference genome (i.e., no mismatches and no gaps are allowed), though this is rather an optional step. Finally, also reads that fall in an in-house curated list of blacklisted regions can be removed. These blacklisted regions comprise common polymorphic CNVs, collapsed repeats, DAC blacklisted regions generated for the ENCODE project and the undefined segment of the reference genome (i.e., the Ns).

* Computing genomic representation

The reference genome is divided in bins of 50kb and the number of reads of the test sample is counted per bin. These read counts are corrected according to the GC contents of the bins with locally weighted scatterplot smoothing (Loess regression). These GC-corrected read counts are then divided by the total sum of all autosomal GC-corrected read counts and multiplied by $10^7$. This is defined as the genomic representations (GR) per bin. On these per-bin GR values a sliding window is applied and the sum of these GR values is computed for all consecutive 100 bins. The windows are each time shifted with 1 bin (i.e., 50 kb). In this way a GR value is obtained per 5Mb window. Similarly, for each autosome also the sum of the per-bin GR values is calculated, to obtain a GR value for each autosome in the test sample.

* Comparison with a reference set

In a reference set of 100 *reference* samples (smaller or larger numbers are also possible) the GR values are computed for all autosomes and for all 5Mb windows as described above. For each autosome and 5Mb window the mean $\mu$ and the standard deviation $\sigma$ of the GR scores are computed over all reference samples. This allows computing a *Z-score* for each window and each autosome $i$ in a test sample, defined as

$$Z_i = \frac{GR_i - \mu_i}{\sigma_i}$$

where $GR_i$ is the GR value in the test sample for window or autosome $i$ and $\mu_i$, $\sigma_i$ the average and standard deviation, respectively, of the GR scores measured in the reference samples for window or autosome $i$.

Based on the 22 Z-scores of the autosomes in a test sample, a *ZZ2 score* is computed for each autosome as

$$2 = \frac{Z_i - \underset{j=1,\dots,22}{\operatorname{median}}(Z_j)}{\underset{j=1,\dots,22}{\operatorname{sd}}(Z_j)}$$

where the Z-score $Z_i$ of autosome $i$ in the test sample is compared with the median and the standard deviation (sd) of the 22 Z-scores obtained for all 22 autosomes in the test sample.

Alternatively, a *ZofZ-score* is computed as

$$Zof\, Z_i = \frac{Z_i - \underset{j=1,\dots,22}{\operatorname{median}}(Z_j)}{\underset{j=1,\dots,22}{\operatorname{mad}}(Z_j)}$$

where the Z-score $Z_i$ of chromosome $i$ in the test sample is compared with the median and the median absolute deviation (mad) of the 22 Z-scores obtained for all 22 autosomes in the test sample. The ZZ2 and ZofZ-scores quantify the deviation of the Z-score of the target autosome from all Z-scores observed in the test sample. This robust version of the Z-of-Z scores does not make any assumptions on the aneuploidy state of the autosome under consideration and the other autosomes.

Based on the Z-scores computed for all 5Mb windows in the test sample, the *BM score* of each autosome $i$ is computed as the median of the Z-scores over all windows of the target autosome:

$$\mathrm{BM}_i = \underset{j=\text{window on i}}{\operatorname{median}}(Z_j)$$

where the median of the Z-scores is computed over all windows $j$ on autosome $i$.

This BM-score reflects the size of the aberration: aneuploidies will result in large BM values, while smaller, segmental CNVs will have less influence on the median of the Z-scores and result in smaller BM-scores. To distinguish high, aberration-related BM scores from augmented BM values due to noise in the data set or the presence of genome-wide instability that could be indicative for cancer, the OM score for an autosome $i$ is computed as the median of the Z-scores of all windows of the other autosomes:

$$OM_i = \underset{j=\text{window not on i}}{\text{median}} |Z_j|$$

where the median is computed over all, absolute Z-scores obtained for 5Mb windows $j$ not located on autosome $i$.

Finally, for each test sample a *quality score (QS)* is calculated as

$$QS = \underset{j}{\text{sd}}(Z_j)$$

with $j$ over all autosomes expect for the 2 autosomes with the highest and the lowest Z-score. This score will identify test samples of poor quality that will result in unreliable aneuploidy calling. A highly elevated QS-score can also hint at DNA samples containing at least a fraction of DNA originating from a tumor.

For each of the above calculated parameters, a threshold value can be defined. Based on standard statistical considerations, one could choose a threshold value of 2, 2.5 or 3. In the context of the Z-score, this means that the chance that the test result is normal (i.e. the obtained GR score is similar as the GR scores for the same region in the reference set) is very unlikely. In order to make a test more specific, one could increase the threshold value. In order to make a test more sensitive, one could decrease the threshold value. These threshold values can be defined for each of the parameters, and can differ for each of the parameters. It is for instance conceivable that threshold values for BM and OM are set to 1, while for Z score and ZZ-score they are set to 3. Also negative threshold values can be used.

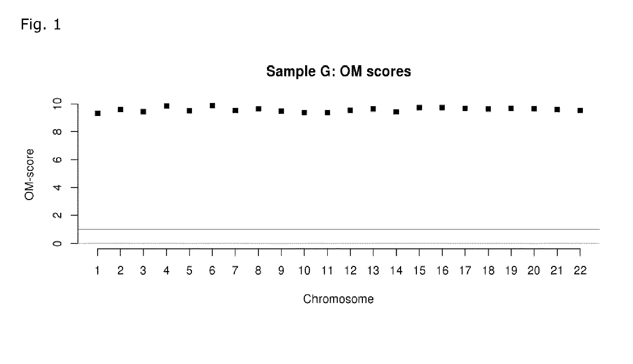

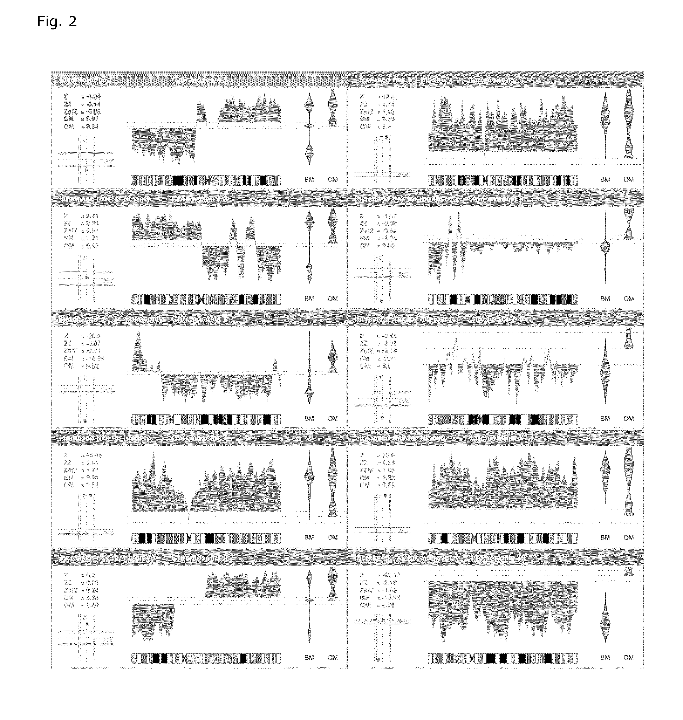

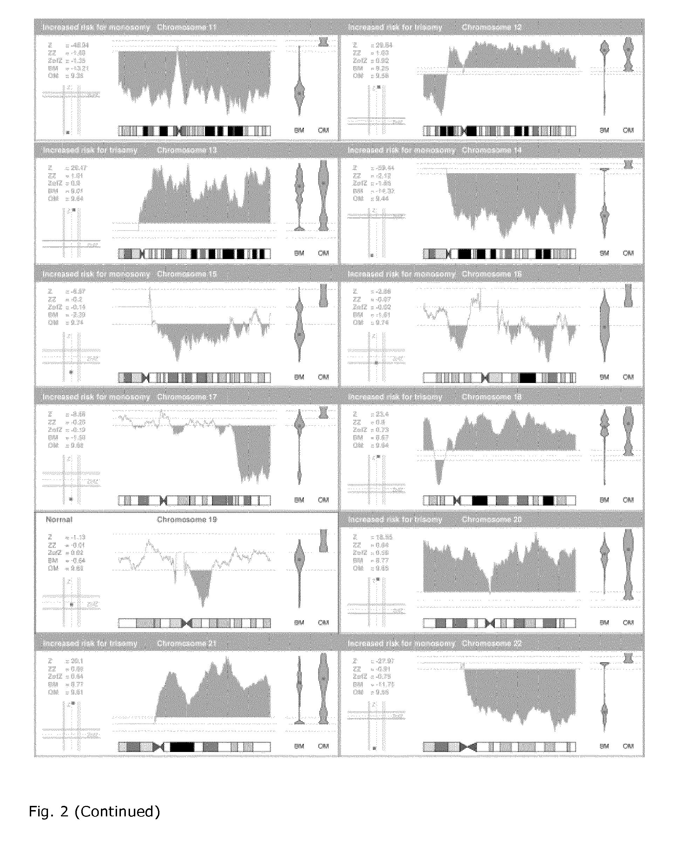

Analysed sample G showed a QS score of 27.526. The plot of the OM values or the autosomes displayed extreme values (Figure 1). Visual inspection of the plots of the individual autosomes confirmed that this sample behaved abnormal (figure 2). This type of overall highly variable patterns in the Z-scores is indicative for genome-wide instability.

It is supposed that the present invention is not restricted to any form of realization described previously and that some modifications can be added to the presented example of fabrication without reappraisal of the appended claims.

**Claims**

1. A method for identifying the presence of tumor-derived cell-free DNA in a mammal, said method comprising:

   - providing the sequences of at least a segment of the nucleic acid molecules contained in a biological sample obtained from a subject, said biological sample comprises cell-free DNA;
   - aligning said obtained sequences to a reference genome;
   - counting the number of reads on a set of chromosomal segments and/or chromosomes thereby obtaining read counts;
   - normalizing said read counts or a derivative thereof into a normalized number of reads;
   - obtaining a first score of said normalized reads and obtaining a collection of scores of said normalized reads, whereby said first score is derived from the normalized reads for a target chromosome or chromosomal segment and whereby said collection of scores is a set of scores derived from the normalized number of reads for a set of chromosomes or chromosome segments that include said target chromosomal segment or chromosome;
   - calculating a parameter p from said first score and said collection of scores, whereby said parameter represents a ratio between

      * said first score, corrected by a summary statistic of said collection of scores, and
      * a summary statistic of said collection of scores; and

   - comparing said parameter by a cutoff value, whereby said cutoff value is prerequisite for the presence or absence of one or more aneuploidies in said target chromosome or chromosome segment which is an indicator of the presence of tumor-derived cell-free DNA.

2. The method according to claim 1, **characterized in that** said number of reads are recalibrated to correct for GC content and/or total number of reads obtained from said sample.

3. The method according to claim 1 or 2, **characterized in that** said normalization occurs via comparison with data obtained from the corresponding chromosomal segments or chromosomes from a reference set.

4. The method according to any one of the claims 1 to 3, **characterized in that** said summary statistic is the mean, median, standard deviation, mean absolute deviation or the median absolute deviation.

5. The method according to anyone of the claims 1 to 4, wherein the sequencing is performed randomly on a segment of the nucleic acid molecules contained in the biological sample.

6. The method according to any one of the previous claims, wherein the biological sample is blood, plasma, serum, urine, transcervical fluid or saliva.

7. The method according to any one of the previous claims, wherein said cutoff value is established using standard statistical considerations, or empirically established by using biological samples

8. The method according to any one of the previous claims whereby said first score is calculated as: $Zi = \frac{GRi - \mu\,ref,i}{\sigma\,ref,i}$ whereby i is a chromosome or chromosomal segment or the target chromosome or target chromosomal segment.

9. The method according to anyone of the previous claims, **characterized in that** said parameter p is calculated as:

$$Z\,of\,Z_i = \frac{Z_i - \underset{j=i,a,b,\ldots}{\mathrm{median}}\left(Z_j\right)}{\underset{j=i,a,b,\ldots}{\mathrm{mad}}\left(Z_j\right)}$$

whereby (Zj) represents a collection of scores that are derived from chromosomes or chromosomal segments i, a, b, .. whereby i corresponds to the target chromosomal segment or chromosome.

10. The method according to any of the previous claims, comprising the calculation of secondary parameters, whereby said secondary parameters are a prerequisite of the amount of said aneuploidy if found present and/or a measure of the quality of the sample.

11. The method according to claim 10, whereby said secondary parameters are compared to a cutoff value.

12. The method according to any one of the claims 10 or 11, said presence or absence of an aneuploidy is determined by the comparison of said parameter to a cutoff value and the comparison of one or more secondary parameters and corresponding cutoff values.

13. The method of anyone of the preceding claim 1 to 12, wherein said aneuploidy comprise whole chromosome aneuploidy, a loss, a gain, an amplification or a deletion of a substantial arm level segment of a chromosome.

14. The method of claim 13, wherein said whole chromosome aneuploidy comprises a gain or a loss as shown in Table 1.

15. The method of claim 14, wherein said target chromosomal segments are substantially arm-level segments comprising a p arm or a q arm of any one or more of chromosomes 1-22, X and Y.

16. The method of claim 15, wherein said target chromosomal segment comprises one or more arms selected from the group consisting of 1q, 3q, 4p, 4q, 5p, 5q, 6p, 6q, 7p, 7q, 8p, 8q, 9p, 9q, 10p, 10q, 12p, 12q, 13q, 14q, 16p, 17p, 17q, 18p, 18q, 19p, 19q, 20p, 20q, 21q, and/or 22q.

17. The method of claim 1 to 14, wherein said aneuploidy comprises an amplification or deletion of one or more arms selected from the group consisting of 1q, 3q, 4p, 4q, 5p, 5q, 6p, 6q, 7p, 7q, 8p, 8q, 9p, 9q, 10p, 10q, 12p, 12q, 13q, 14q, 16p, 17p, 17q, 18p, 18q, 19p, 19q, 20p, 20q, 21q, 22q.

18. The method of claim 1 to 17, wherein said chromosomal segments are segments that comprise a region and/or a gene shown in Table 3 and/or Table 5 and/or Table 4 and/or Table 6.

19. The method of claim 1 to 17, wherein said aneuploidy comprises an amplification of a region and/or a gene shown in Table 3 and/or Table 5.

20. The method of claim 1 to 17, wherein said aneuploidy comprises a deletion of a region and/or a gene shown in Table 4 and/or Table 6.

21. The method of claim 1 to 20, wherein said chromosome segments are segments known to contain one or more oncogenes and/or one or more tumor suppressor genes.

22. The method of claim 1 to 17, wherein said aneuploidy comprises an amplification of one or more regions selected from the group consisting of 20Q13, 19q12, 1q21-1q23, 8p11-p12, MYC, ERBB2 (EFGR), CCND1 (Cyclin D1), FGFR1, FGFR2, HRAS, KRAS, MYB, MDM2, CCNE, NRAS, MET, ERBB1, CDK4, MYCB, ERBB2, AKT2, MDM2, BRAF, ARAF, CRAF, PIK3CA, AKT1, PTEN, STK11, MAP2K1, ALK, ROS1, CTNNB1, TP53, SMAD4, FBX7, FGFR3, NOTCH1, ERBB4 and CDK4, and the like.

23. The method of any one of claim 1 to 22, wherein said cancer is a cancer selected from the group consisting of leukemia, ALL, brain cancer, breast cancer, colorectal cancer, dedifferentiated liposarcoma, esophageal adenocarcinoma, esophageal squamous cell cancer, GIST, glioma, HCC, hepatocellular cancer, lung cancer, lung NSC, lung SC, medulloblastoma, melanoma, MPD, myeloproliferative disorder, cervical cancer, ovarian cancer, prostate cancer, and renal cancer.

24. The method of any one of claim 1 to 23, wherein detection of aneuploidies indicates a positive result and said method further comprises prescribing, initiating, and/or altering treatment of a human subject from whom the test sample was taken.

25. The method of claim 24, wherein said prescribing, initiating, and/or altering treatment of a human subject from whom the test sample was taken comprises prescribing and/or performing further diagnostics to determine the presence and/or severity of a cancer.

26. The method of claim 25, wherein said further diagnostics comprise screening a sample from said subject for a biomarker of a cancer, and/or imaging said subject for a cancer.

27. A computer program product comprising a computer readable medium encoded with a plurality of instructions for controlling a computing system to perform an operation for performing the analysis the presence of a cancer and/or an increased risk of a cancer in a mammal in a biological sample obtained from a subject, wherein the biological sample includes nucleic acid molecules, the operation comprising the steps of

- receiving the sequences of at least a segment of the nucleic acid molecules contained in a biological sample obtained from subject, said biological sample comprises cell-free DNA;
- aligning said obtained sequences to a reference genome;
- counting the number of reads on a set of chromosomal segments and/or chromosomes thereby obtaining the read counts;
- normalizing said read counts or a derivative thereof into a normalized number of reads;
- obtaining a first score of said normalized reads and obtaining a collection of scores of said normalized reads, whereby said first score is derived from the normalized reads for a target chromosome or chromosomal segment and whereby said collection of scores is a set of scores derived from the normalized reads for a set of chromosomes or chromosomal segments that include said target chromosomal segment or chromosome;
- calculating a parameter p from said first score and said collection of scores, whereby said parameter represents a ratio between

    * said first score, corrected by a summary statistic of said collection of scores, and
    * a summary statistic of said collection of scores; and

- comparing said parameter by a cutoff value, whereby said cutoff value is prerequisite for the presence or absence of one or more aneuploidies in said target chromosome or chromosome segment which is an indicator of the presence and/or increased risk of cancer.

28. Computer program product according to claim 27, further comprising operations for calculating one or more secondary parameters, whereby said secondary parameters are a prerequisite of the intensity of said aneuploidy if found present and/or a measure of quality of the sample.

**29.** A kit comprising a computer program product according to any one of the claims 27 or 28 and a protocol for obtaining the sequences of at least a portion of the nucleic acid molecules contained in a biological sample, said biological sample comprises cell-free DNA.

**30.** A report, comprising an estimation of the presence or absence of a chromosomal aneuploidy in a subject, said report comprises parameter p, one or more secondary parameters and comparison to a cutoff value as defined in any one of the claims 1 to 27 and a visualization of said reads per chromosome.

Fig. 1

Sample G: OM scores

Fig. 2

Fig. 2 (Continued)

Europäisches
Patentamt

European
Patent Office

Office européen
des brevets

## EUROPEAN SEARCH REPORT

Application Number

EP 15 17 6404

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2014/190286 A2 (SEQUENOM INC [US]) 27 November 2014 (2014-11-27) * page 174, lines 1-10; claim 1 * | 1-30 | INV. C12Q1/68 G06F19/18 G06F19/20 |
| X | WO 2013/109981 A1 (SEQUENOM INC [US]) 25 July 2013 (2013-07-25) * page 135, line 14 - line 29; claims 30-60 * * page 136, line 10 - page 137 * * page 82, lines 1-15 * | 1-30 | G06F19/22 |
| X | WO 2014/015319 A1 (VERINATA HEALTH INC [US]) 23 January 2014 (2014-01-23) * page 165; claim 1 * | 1-30 | |
| X | WO 2014/014497 A1 (VERINATA HEALTH INC [US]; RAVA RICHARD P [US]; CHINNAPPA MANJULA [US];) 23 January 2014 (2014-01-23) * example 15 * * page 304 - page 304 * | 1-30 | |
| | | | TECHNICAL FIELDS SEARCHED (IPC) |
| X | WO 2015/061359 A1 (VERINATA HEALTH INC [US]) 30 April 2015 (2015-04-30) * paragraph [0292]; claim 1 * * paragraph [0223] * * paragraph [0174] * * paragraph [0155] - paragraph [0156] * * paragraph [0200] - paragraph [0201] * | 1-30 | C12Q G06F |

The present search report has been drawn up for all claims

| Place of search | Date of completion of the search | Examiner |
|---|---|---|
| The Hague | 12 January 2016 | Gabriels, Jan |

## ANNEX TO THE EUROPEAN SEARCH REPORT
## ON EUROPEAN PATENT APPLICATION NO.

EP 15 17 6404

This annex lists the patent family members relating to the patent documents cited in the above-mentioned European search report.
The members are as contained in the European Patent Office EDP file on
The European Patent Office is in no way liable for these particulars which are merely given for the purpose of information.

12-01-2016

| Patent document cited in search report | | Publication date | Patent family member(s) | | Publication date |
|---|---|---|---|---|---|
| WO 2014190286 | A2 | 27-11-2014 | AU | 2014268377 A1 | 12-11-2015 |
| | | | CA | 2910205 A1 | 27-11-2014 |
| | | | WO | 2014190286 A2 | 27-11-2014 |
| WO 2013109981 | A1 | 25-07-2013 | AU | 2013209499 A1 | 14-08-2014 |
| | | | CA | 2861856 A1 | 25-07-2013 |
| | | | EP | 2805280 A1 | 26-11-2014 |
| | | | HK | 1202672 A1 | 02-10-2015 |
| | | | JP | 2015513392 A | 14-05-2015 |
| | | | WO | 2013109981 A1 | 25-07-2013 |
| WO 2014015319 | A1 | 23-01-2014 | US | 2014038830 A1 | 06-02-2014 |
| | | | WO | 2014015319 A1 | 23-01-2014 |
| WO 2014014497 | A1 | 23-01-2014 | AU | 2013204615 A1 | 06-02-2014 |
| | | | CA | 2878246 A1 | 23-01-2014 |
| | | | EP | 2875149 A1 | 27-05-2015 |
| | | | EP | 2877594 A1 | 03-06-2015 |
| | | | WO | 2014014497 A1 | 23-01-2014 |
| | | | WO | 2014014498 A1 | 23-01-2014 |
| WO 2015061359 | A1 | 30-04-2015 | NONE | | |

EPO FORM P0459

For more details about this annex : see Official Journal of the European Patent Office, No. 12/82

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20130034546 A
- US 20130310263 A

**Non-patent literature cited in the description**

- **LIN et al.** *Cancer Res,* 2008, vol. 68, 664-673
- **GEORGE et al.** *PLoS ONE,* 2007, vol. 2, e255
- **DEMICHELIS et al.** *Genes Chromosomes Cancer,* 2009, vol. 48, 366-380
- **BEROUKHIM et al.** *Nature,* 2010, vol. 463 (7283), 899-905
- **PARK et al.** *Clinical Breast Cancer,* 2008, vol. 8, 392-401
- **VARMUS H.** *Ann Rev Genetics,* 1984, vol. 18, 553-612
- **WATSON et al.** Molecular Biology of the Gene. Benjamin/Cummings Publishing Co, 1987
- **HOWE et al.** *Proc Natl Acad Sci (USA),* 1990, vol. 87, 5883-5887
- **RYGAARD et al.** *Cancer Res,* 1990, vol. 50, 5312-5317
- **BOWCOCK et al.** *Am J Hum Genet,* 1990, vol. 46, 12
- **CARAMAZZA et al.** *Eur J Hematol,* 2010, vol. 84, 191-200
- **EISENMANN et al.** *Oncogene,* 2009, vol. 28, 3429-3441
- **ANGELONI D.** *Briefings Functional Genomics,* 2007, vol. 6, 19-39
- **FONATSCH C.** *Gene Chromosomes Cancer,* 2010, vol. 49, 497-508
- **BENJAMINI et al.** *Nucleic Acid Research,* 2012